(19) Europäisches Patentamt
European Patent Office
Office européen des brevets

(11) **EP 2 132 593 B1**

(12) **EUROPÄISCHE PATENTSCHRIFT**

(45) Veröffentlichungstag und Bekanntmachung des Hinweises auf die Patenterteilung:
**20.06.2012 Patentblatt 2012/25**

(21) Anmeldenummer: **08734912.2**

(22) Anmeldetag: **31.03.2008**

(51) Int Cl.:
**A61B 3/04** (2006.01)  **G02C 7/02** (2006.01)

(86) Internationale Anmeldenummer:
**PCT/EP2008/002557**

(87) Internationale Veröffentlichungsnummer:
**WO 2008/119542 (09.10.2008 Gazette 2008/41)**

(54) **SUBJEKTIVE SIMULATION DER ABBILDUNGSEIGENSCHAFTEN VON SPORTBRILLEN**

SUBJECTIVE SIMULATION OF THE IMAGING PROPERTIES OF SPORTS GLASSES

SIMULATION SUBJECTIVE DES PROPRIÉTÉS DE REPRÉSENTATION DE LUNETTES DE SPORT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **02.04.2007 DE 102007015908**

(43) Veröffentlichungstag der Anmeldung:
**16.12.2009 Patentblatt 2009/51**

(73) Patentinhaber: **Rodenstock GmbH**
**80469 München (DE)**

(72) Erfinder:
• **ALTHEIMER, Helmut**
**87650 Baisweil-Lauchdorf (DE)**
• **BECKEN, Wolfgang**
**81541 München (DE)**
• **ESSER, Gregor**
**81735 München (DE)**
• **SEIDEMANN, Anne**
**80337 München (DE)**
• **WELK, Andrea**
**81547 München (DE)**
• **UTTENWEILER, Dietmar**
**82057 Icking (DE)**

(74) Vertreter: **Müller-Boré & Partner**
**Patentanwälte**
**Grafinger Straße 2**
**81671 München (DE)**

(56) Entgegenhaltungen:
**DE-A1-102005 057 533    US-A- 5 742 375**

**Beschreibung**

Die vorliegende Erfindung betrifft die Untersuchung und Bestimmung der Verträglichkeit einer Brille mit großem Fassungsscheibenwinkel bzw. mit starker Durchmuschelung, insbesondere einer Sportbrille, für einen Brillenträger.

Spezielle Sportbrillen mit Korrektionswirkung, die sowohl mit Einstärken- als auch mit Gleitsichtgläsern verglast werden können, werden am Markt immer beliebter und immer mehr Brillenglashersteller bieten speziell ausgelegte und berechnete Sportbrillengläser an. Charakteristisch für Sportbrillen ist die starke Durchmuschelung der Fassungsränder und die relativ hohe Mittelteildurchbiegung der Fassungen, d.h. bei den Sportbrillen sitzen die Brillengläser nicht gerade vor den Augen, sondern sind um ca. 10°-25° schiefgestellt und müssen mit starken Durchbiegungen (Basiskurve 6-8 dpt) gefertigt werden ("wrap around"). Schiefgestellte Brillengläser mit und ohne starker Durchbiegung führen, selbst wenn bei der Berechnung der Gläser der Sitz vor dem Auge berücksichtigt wird, bei den Brillenträgern häufig zu sog. asthenopischen Beschwerden mit geänderten Raumwahrnehmungen und nicht selten zu vollkommener Unverträglichkeit der Brillen.

Für die sensorische Fusion unter besonderen Abbildungseigenschaften, wie sie beispielsweise bei Sportbrillen oft auftreten, sind die physiologischen Schwellenwerte individuell von Brillenträger zu Brillenträger sehr unterschiedlich. Eine allgemein gültige Korrelation zwischen Glasstärke, Fassungsscheibenwinkel und Verträglichkeit bzw. Unverträglichkeit ist nicht möglich und würde insbesondere den individuellen Anforderungen einzelner Brillenträger nicht gerecht. Deshalb ist es bisher kaum möglich, bei jedem individuellen Brillenträger Vorhersagen über die Verträglichkeit einer Brille, insbesondere einer Sportbrille mit Korrektionswirkung zu machen.

Es ist somit Aufgabe der vorliegenden Erfindung, ein Verfahren und eine Vorrichtung bereitzustellen, um eventuelle Verträglichkeitsprobleme bei einem Brillenträger bereits im Voraus aufzudecken. Diese Aufgabe wird durch Verfahren mit den in den Ansprüchen 1, 4, 8 oder 9, ein Computersystem mit den in den Ansprüchen 6 und 7, ein Computerprogrammprodukt mit den im Anspruch 10 sowie Gläsersätzen mit den in den Ansprüchen 11 oder 14 angegebenen Merkmalen gelöst. Bevorzugte Ausführungsformen sind Gegenstand der abhängigen Ansprüche.

Somit stellt die Erfindung in einem Aspekt ein Verfahren zur insbesondere nicht-diagnostischen Untersuchung der Verträglichkeit einer Brille mit großem Fassungsscheibenwinkel und/oder mit starker Durchmuschelung, insbesondere einer Sportbrille, für einen Brillenträger bereit, welches umfasst:

- Entwerfen zumindest eines Brillenglases bzw. eines Designs für zumindest ein Brillenglas der Brille für den Brillenträger;
- Ermitteln einer Vergrößerung und/oder einer Verzerrung des zumindest einen Brillenglases für zumindest eine Blickrichtung des Brillenträgers vorzugsweise in einer individuellen Gebrauchsstellung;
- Auswählen zumindest eines afokalen Demonstrationsglases mit einer bestimmten Eigenvergrößerung aus einer insbesondere vorgefertigten Serie von afokalen Demonstrationsgläsern, d.h. zwei oder mehr afokale Demonstrationsgläser, mit zumindest teilweise unterschiedlichen Eigenvergrößerungen abhängig von der ermittelten Vergrößerung und/oder Verzerrung des zumindest einen Brillenglases; und
- Vorhalten des ausgewählten afokalen Demonstrationsglases in einer Demonstrationsstellung vor das entsprechende Auge des Brillenträgers.

Das Entwerfen des zumindest einen Brillenglases bedeutet dabei ein Erstellen eines Entwurfes bzw. Designs für ein Brillenglas, also ein Festlegen des gewünschten Brillenglas in seinen geometrischen Flächenformen und -positionen und/oder seinen optischen Eigenschaften zumindest derart, dass es möglich ist, für zumindest eine Blickrichtung des Brillenträgers die zu erwartende Vergrößerung und/oder Verzerrung des Brillenglases für den Brillenträger auf Basis dieses Entwurfes bzw. Designs zu ermitteln bzw. vorherzusagen. Das Entwerfen des Brillenglases bzw. des Designs für das Brillenglas umfasst dabei insbesondere noch nicht die tatsächliche Herstellung des Glases z.B. aus einem Rohling bzw. Halbfertigprodukt. Der Entwurf bzw. das Design des Brillenglases umfasst damit insbesondere lediglich Daten auf deren Basis das Brillenglas zumindest teilweise hergestellt werden könnte. Das Entwerfen des Brillenglases bzw. des Designs für das Brillenglas bildet dabei insbesondere zumindest einen Teil eines Optimierungs- bzw. Berechnungsverfahrens für die Herstellung eines Brillenglas. Damit stellt der Entwurf oder das "Design" eines Brillenglases im Sinne der Erfindung insbesondere einen Datensatz dar, der Form und/oder Position bzw. Verlauf von Vorderfläche und Rückfläche des Brillenglases und/oder die durch die Vorder- und Rückfläche bewirkten optischen Eigenschaften des Brillenglases soweit festlegen, dass daraus eine Vergrößerung und/oder Verzerrung des Brillenglases für den Brillenträger in zumindest einer Blickrichtung des Brillenträgers bestimmt werden können.

Durch das Vorhalten des ausgewählten afokalen Demonstrationsglases in der Demonstrationsstellung vor das entsprechende Auge des Brillenträgers wird eine von der Eigenvergrößerung und der Demonstrationsstellung abhängige Vergrößerung und/oder Verzerrung für den Brillenträger erreicht, die dem Brillenträger einen Eindruck dessen liefert, was er als Seheindruck im Hinblick auf die Vergrößerung und/oder Verzerrung von der gewünschten Brille mit großem Fassungsscheibenwinkel und/oder mit starker Durchmuschelung zu erwarten hat.

Damit lassen sich in besonders einfacher Weise optische Effekte, wie z.B. Verzerrung und/oder Eigenvergrößerung einer Brille mit großem Fassungsscheibenwinkel und/oder mit starker Durchmuschelung einem Brillenträger präsentieren, bevor die Brille bestellt oder gefertigt ist. Vorzugsweise lässt sich damit die Vergrößerung und/oder die Verzerrung des zumindest einen Brillenglases in der individuellen Gebrauchsstellung zumindest annähernd demonstrieren. So resultieren eventuelle Unverträglichkeiten beispielsweise von Sportbrillen durch manche Benutzer unter anderem in veränderten Abbildungseigenschaften der schiefgestellten Sportgläser mit starker Durchbiegung. Die Gläser besitzen stärkere Vergrößerungen, die Verteilung der Abbildungsfehler nasal/temporal ist oft sehr unsymmetrisch und es treten zudem sehr starke, nasal und temporal unterschiedlich große, Verzerrungen auf. Dies führt sowohl monokular als auch binokular zu Beeinträchtigungen der visuellen Wahrnehmungen mit den möglichen Folgen von verfälschter Raumwahrnehmung, gestörter Entfernungseinschätzung, Fusionsproblemen, Doppelbildern, Kopfschmerzen, allgemeines Unwohlsein, usw..

Mittels der vorliegenden Erfindung können solche optische Eigenschaften von Sportbrillen in einfacher Weise nachempfunden und die resultierenden Probleme in besonders effizienter Weise auf die individuelle Situation des Brillenträgers abgestimmt bzw. bereits im Vorfeld untersucht bzw. bestimmt werden. Die Resultate der erfindungsgemäßen Untersuchungs- bzw. Bestimmungsverfahren fließen dabei in das Herstellungsverfahren eines Brillenglases ein. Dadurch können einerseits Fehlproduktionen bzw. Rückläufer von Brillengläsern aufgrund von Unverträglichkeiten vermieden oder zumindest reduziert werden, was zu einer Erhöhung der Ausbeute an Brillengläsern bei der Herstellung führt. Andererseits kann damit der Toleranzbereich für die Vergrößerung bzw. Verzerrung in Bezug auf die individuelle Verträglichkeit zugunsten einer besseren Optimierung anderer evtl. konkurrierender optischer oder geometrischer Parameter, wie z.B. der Refraktionskorrektur oder der individuellen Kopfform, besser ausgenutzt werden.

Insbesondere wird hierbei das zumindest eine ausgewählte Demonstrationsglas in der Demonstrationsstellung in das Blickfeld zumindest desjenigen Auges gebracht, für welches das zumindest eine Brillenglas der Brille, wie z.B. einer Sportbrille, entworfen wurde. Damit werden in besonders einfacher Weise die individuell zu erwartenden optischen Eigenschaften des geplanten Brillenglases für den Brillenträger erfahrbar nachempfunden. Dabei wird das afokale Demonstrationsglas gerade so ausgewählt, dass die resultierende optische Wirkung der entsprechenden optischen Wirkung des geplanten Brillenglases insbesondere für die zumindest eine Blickrichtung möglichst nahe kommt. Insbesondere lassen sich beispielsweise die zu erwartende Vergrößerung des zumindest einen Brillenglases durch die Auswahl einer geeigneten Eigenvergrößerung des afokalen Demonstrationsglases nachempfinden, während eine Verzerrung vorzugsweise durch eine Verkippung des geeignet ausgewählten Demonstrationsglases erzeugt wird.

Die durch ein Verkippen des Demonstrationsglases bewirkte Verzerrung hängt insbesondere vom Verkippungswinkel und der Eigenvergrößerung des ausgewählten Demonstrationsglases und damit von der für die Auswahl des Demonstrationsglases herangezogenen Vergrößerung des zumindest einen Brillenglases für die zumindest eine Blickrichtung ab. Andererseits lässt sich ein Einfluss der Verkippung auf die Vergrößerungswirkung des afokalen Demonstrationsglases in der Demonstrationsstellung für den Brillenträger bereits bei der Auswahl des Demonstrationsglases aus der Serie von Demonstrationsgläsern berücksichtigen. So wird insbesondere eine Erhöhung der Vergrößerung des afokalen Demonstrationsglas für die zumindest eine Blickrichtung aufgrund der Verkippung in der Demonstrationsstellung bereits durch eine Auswahl eines afokalen Demonstrationsglases mit einer geeignet niedrigeren Eigenvergrößerung berücksichtigt.

Die Erfindung betrifft somit insbesondere ein Verfahren zur Untersuchung der Verträglichkeit einer Brille mit großem Fassungsscheibenwinkel und/oder mit starker Durchmuschelung für einen Brillenträger, umfassend:

- Entwerfen zumindest eines Brillenglases der Brille für den Brillenträger;
- Ermitteln einer Vergrößerung und/oder Verzerrung des zumindest einen Brillenglases für zumindest eine Blickrichtung des Brillenträgers;
- Bereitstellen einer Serie von afokalen Demonstrationsgläsern mit zumindest teilweise unterschiedlichen Eigenvergrößerungen;
- Auswählen desjenigen afokalen Demonstrationsglases aus der Serie von afokalen Demonstrationsgläsern, das in einer Demonstrationsstellung vor dem entsprechenden Auge des Brillenträgers für die zumindest eine Blickrichtung des Brillenträgers zu einer Vergrößerung und/oder Verzerrung führt, die der ermittelten Vergrößerung bzw. Verzerrung des zumindest einen Brillenglases am nächsten kommt;
- Vorhalten des ausgewählten afokalen Demonstrationsglases in der Demonstrationsstellung von das entsprechende Auge des Brillenträgers.

Dabei ist die Erfindung nicht auf bestimmte Werte des Fassungsscheibenwinkels oder der Durchmuschelung der zu bestellenden Brille beschränkt. So beziehen sich die Bezeichnungen "großer Fassungsscheibenwinkel" und "starke Durchmuschelung" vorzugsweise auf alle Fassungsscheibenwinkel bzw. Durchmuschelungen, die groß genug sind, um für einen Brillenträger eventuell zu den genannten Problemen und Unverträglichkeiten führen zu können. So könnten sich bereits bei für typische Sportbrillen relativ kleinen Fassungsscheibenwinkeln oder Durchmuschelungen für manche Brillenträger bereits Unverträglichkeiten beispielsweise aufgrund von Vergrößerungen der Brillengläser und/ oder aufgrund von Verzerrungen oder deren Inhomogenität bzw. Verteilung (z.B. nasal - temporal), d.h. aufgrund von Veränderungen der Vergrößerung und/oder Verzerrung bei Blickbewegung, insbesondere zu sogenannten asthenopischen Beschwerden führen. Auch solche "relativ kleinen" Fassungsscheibenwinkel bzw. Durchmuschelungen sind von der Erfindung erfasst.

Vorzugsweise wird zu jeder Blickrichtung oder zumindest für eine Vielzahl von Blickrichtungen analytisch die Vergrößerung und/oder die Verzerrung des zumindest einen Brillenglases auf Basis des Designs bzw. Entwurfes des Brillenglases berechnet. Als Verzerrung wird dabei insbesondere die Differenz einer kleinsten und einer größten axialen Vergrößerung verstanden. Insbesondere erhält man analog zur astigmatischen Abbildung zu jedem Hauptstrahl eine kleinste axiale Vergrößerung $N_1$ und eine größte axiale Vergrößerung $N_2$, deren Achsen aufeinander senkrecht stehen und die auch als Hauptvergrößerungen bezeichnet werden. Die Differenz dieser beiden Hauptvergrößerungen $N_2-N_1$ wird als Verzerrung und der Mittelwert der Hauptvergrößerungen $(N_1+N_2)/2$ als Vergrößerung bezeichnet. Plusgläser vergrößern, Minusgläser verkleinern. Entsprechendes gilt bei der Bestimmung bzw. Festlegung bzw. Ermittlung eines Vergrößerungs- bzw. Verzerrungsunterschieds zwischen beiden Augen des Brillenträgers. Dabei lässt sich für zwei korrespondierende Blickrichtungen bzw. Hauptstrahlen des rechten bzw. linken Auges in analoger Weise zu jeder Achslage ein Unterschied der Vergrößerungen für beide Augen bestimmen. Dabei ergeben sich zwei senkrecht zueinander stehende Achsen für die der Unterschied der Vergrößerungen minimal bzw. maximal wird. Entsprechend wird vorzugsweise die Differenz dieser beiden Unterschiede der Vergrößerungen als Verzerrungsunterschied bezeichnet, während ihr Mittelwert als Vergrößerungsunterschied bezeichnet wird.

Zwar ließe sich der Verzerrung ebenso wie dem Verzerrungsunterschied über die Orientierung der beiden Achsen auch eine Richtung zuordnen, in der folgenden Beschreibung wird als Verzerrung bzw. Verzerrungsunterschied im Sinne der Erfindung aber, soweit nicht explizit etwas anderes erwähnt ist, stets nur dessen Wert als Differenz der Werte für die maximale und minimale axiale Vergrößerung bzw. für den maximalen und minimalen axialen Unterschied der Vergrößerung des rechten und linken Auges, also im Falle der Verzerrung der Betrag der Differenz der Hauptvergrößerungen verstanden. Die Verzerrungsrichtung fällt im Allgemeinen nicht mit der Richtung eines Hauptschnittes der astigmatischen Abbildung zusammen. Nur bei zentrierten, nicht prismatischen Einstärkengläsern entlang einer Symmetrieebene und bei rotationssymmetrischen Brillengläsern fallen die Richtungen der Hauptvergrößerungen und der Hauptschnittsbrechwerte zusammen. Während der astigmatische Restfehler und der Refraktionsfehler die Bildschärfe beeinflussen, bestimmen die Vergrößerung und die Verzerrung die geometrische Größe und Gestalt des Bildes. Die physiologischen Auswirkungen der Verzerrung betreffen unter anderem die anamorphotische Verzerrung.

Die Vergrößerung eines Brillenglases für den Brillenträger ist insbesondere als Quotient der Netzhautbildgröße mit Brillenglas zur Netzhautbildgröße ohne Brillenglas zu verstehen. Bei einer unscharfen Abbildung auf der Netzhaut lässt sich für die Bestimmung der Vergrößerung des Brillenglases für den Brillenträger eine entsprechende Näherung auf Basis des Hauptstrahls durchführen. Für weitere Details zu bevorzugten Methoden der Bestimmung einer Vergrößerung eines Brillenglases für einen Brillenträger, insbesondere für das Konzept des Hauptstrahls, wird ausdrücklich auf das Lehrbuch von Heinz Diepes und Ralf Blendowske "Optik und Technik der Brille", Ausgabe 2002, Optische Fachveröffentlichung GmbH, Heidelberg Bezug genommen. Insbesondere wenn das entworfene Brillenglas zur Korrektion eines Refraktionsdefizits eines Auges des Brillenträgers ausgelegt ist, lässt sich die Vergrößerung und/oder die Verzerrung des Brillenglases für den Brillenträger besonders effizient über eine lokale Wellenfront, die einem zu einer bestimmten Blickrichtung gehörigen Hauptstrahl zugeordnet ist, ermitteln. Hierzu wird ausdrücklich auf die Druckschrift DE 10 2005 057 533 A1 Bezug genommen.

Vorzugsweise umfasst das Verfahren außerdem ein Bestimmen eines Verkippungswinkels für das zumindest eine ausgewählte afokale Demonstrationsglas abhängig von der ermittelten Vergrößerung und/oder Verzerrung des zumindest einen Brillenglases, wobei das Vorhalten des zumindest einen ausgewählten Demonstrationsglases ein Vorhalten des Demonstrationsglases unter dem bestimmten Verkippungswinkel umfasst. Insbesondere wird dabei der Verkippungswinkel derart bestimmt, dass das ausgewählte afokalen Demonstrationsglas in der Demonstrationsstellung vor dem entsprechenden Auge des Brillenträgers für die zumindest eine Blickrichtung des Brillenträgers zu einer Vergrößerung und/oder Verzerrung führt, die der ermittelten Vergrößerung bzw. Verzerrung des zumindest einen Brillenglases am nächsten kommt.

Vorzugsweise charakterisiert der Verkippungswinkel zumindest teilweise die Demonstrationsstellung bzw. -position. Die Demonstrationsstellung wird dabei vorzugsweise relativ zu einer Demonstrationsausgangsstellung charakterisiert, d.h. die Demonstrationsstellung ist um den ermittelten bzw. bestimmten Verkippungswinkel gegen die Demonstrationsausgangsstellung verkippt. Vorzugsweise stellt die Demonstrationsausgangsstellung eine für den Brillenträger individuell und insbesondere relativ zu dessen Augen und/oder relativ zu einer eventuell von ihm getragenen gewöhnlichen Brille festgelegte Gebrauchsstellung mit 0˚ Fassungsscheibenwinkel und 0˚ Vorneigung dar. Die Demonstrationsstellung wird dabei vorzugsweise zumindest teilweise durch den Verkippungswinkel relativ zur Demonstrationsausgangsstellung derart charakterisiert, dass der Verkippungswinkel einen Fassungsscheibenwinkel und/oder eine Vorneigung zumindest teilweise festlegt.

In einer bevorzugten Ausführungsform wird durch ein Verkippen um eine vertikale Achse der Fassungsscheibenwinkel auf den Verkippungswinkel eingestellt. Insbesondere durch ein verkipptes Vorhalten des in Abhängigkeit von der ermittelten Vergrößerung und/oder Verzerrung ausgewählten Demonstrationsglases kann dem Brillenträger beispielsweise die Verzerrung des gewünschten Brillenglases und/oder ein Verlauf der Vergrößerung, d.h. verschiedenen Vergrößerungen bei verschiedenen Blickrichtungen, zumindest teilweise demonstriert werden. Somit wird vorzugsweise der Verkippungswinkel bzw. der Fassungsscheibenwinkel einer Demonstrationsfassung insbesondere dann in Abhängigkeit von der ermittelten Vergrößerung bestimmt, wenn als Vergrößerung die Werte der Vergrößerung des zumindest einen Brillenglases für verschiedene Blickrichtungen ermittelt wurden.

Vorzugsweise wird das zumindest eine Demonstrationsglas zusätzlich zu einer gewohnten Brille des Brillenträger in der Demonstrationsstellung und insbesondere unter dem bestimmten Verkippungswinkel vor den Augen des Brillenträgers angeordnet.

Dabei trägt der Brillenträger vorzugsweise seine gewohnte Brille, die insbesondere zumindest in der Durchblickrichtung bzw. Hauptblickrichtung die erforderliche Korrektionswirkung, d.h. die erforderlichen Rezeptwerte, wie z.B. die verschriebenen dioptrischen und/oder astigmatischen Wirkungen aufweist. Damit können in besonders einfacher Weise beispielsweise die erforderlichen individuellen Korrektionswirkungen, wie z.B. eine erwünschte dioptrische und/oder prismatische Wirkung und/oder ein erwünschter Astigmatismus und/oder eine zu korrigierende Aniseikonie, etc., durch die gewohnte Brille erreicht werden, während lediglich die zusätzlich beispielsweise durch einen evtl. erhöhten Fassungsscheibenwinkel, einer evtl. erhöhten Durchbiegung bzw. Durchmuschelung, eine evtl. größere Mittendicke, etc. der geplanten Sportbrille hervorgerufenen Vergrößerungen und/oder Verzerrungen durch das zumindest eine ausgewählte Demonstrationsglas in der Demonstrationsstellung nachempfunden bzw. simuliert bzw. demonstriert werden. Das afokale Demonstrationsglas weist somit vorzugsweise im wesentlichen keine dioptrische und/oder astigmatische Wirkung auf, so dass die gesamte dioptrische und/oder astigmatische Wirkung durch das Demonstrationsglas im wesentlichen nicht verändert und lediglich von der gewohnten Brille des Brillenträgers bestimmt wird. Andererseits weist unter bevorzugten Bedingungen die gewohnte Brille des Brillenträgers keine wesentlichen Verzerrungsunterschiede in korrespondierenden Durchblickstellen auf den nasalen und temporalen Hälften der Brillengläser auf.

Die Erfindung stellt sich besonders in den Fällen als vorteilhaft heraus, in denen die Eigenvergrößerung und/oder die Verzerrung der zu entwerfenden Sportbrille und/oder ihre Vergrößerung insbesondere in einer individuellen Gebrauchsstellung erheblich größer ist als die Eigenvergrößerung und/oder die Verzerrung der gewohnten Brille des Brillenträgers und/oder ihre Vergrößerung insbesondere in der gewohnten individuellen Gebrauchsstellung. Besonders in diesen Fällen ist eine vorherige Untersuchung bzw. Bestimmung der Verträglichkeit der zu entwerfenden Sportbrille wünschenswert. Hier lässt sich vorzugsweise die Vergrößerung und/oder die Verzerrung der gewohnten Brille des Brillenträgers gegenüber der durch das ausgewählte afokale Demonstrationsglas in der Demonstrationsstellung eingeführte Vergrößerung bzw. Verzerrung vernachlässigen.

In einer weiteren bevorzugten Ausführungsform erfolgt das Auswählen des zumindest einen afokalen Demonstrationsglases und/oder das Bestimmen der Demonstrationsstellung, insbesondere des Verkippungswinkels, in Abhängigkeit von optischen Eigenschaften und/oder einer individuellen Gebrauchsstellung der vom Brillenträger während des Vorhaltens des Demonstrationsglases getragenen gewohnten Brille. Dabei erfolgt das Auswählen insbesondere in Abhängigkeit von individuellen Abbildungseigenschaften der gewohnten Brille. Somit können eventuell bereits in der gewohnten Brille vorhandene, insbesondere unerwünschte Vergrößerungen und/oder Verzerrungen berücksichtigt werden, um durch eine geeignete Auswahl des zumindest einen Demonstrationsglases und/oder durch eine geeignete Bestimmung der Demonstrationsstellung, insbesondere des Verkippungswinkels, den tatsächlichen Abbildungseigenschaften der geplanten Brille (z.B. Sportbrille), insbesondere im Hinblick auf die Vergrößerung und/oder Verzerrung, durch das Zusammenwirken des Demonstrationsglases mit der vom Brillenträger getragenen gewohnten Brille möglichst nahe zu kommen. Dies ist besonders vorteilhaft, wenn die gewohnte Brille beispielsweise bereits einen Fassungsscheibenwinkel und/oder eine Vorneigung und/oder eine Durchmuschelung aufweist.

Vorzugsweise wird das zumindest eine Demonstrationsglas mittels einer Demonstrationsfassung in der Demonstrationsstellung und insbesondere unter dem bestimmten Verkippungswinkel vor den Augen des Brillenträgers angeordnet.

Vorzugsweise wird eine Demonstrationsfassung mit zumindest einem einstellbaren bzw. veränderbaren Fassungsscheibenwinkel verwendet. Das Bestimmen eines Verkippungswinkels umfasst dabei vorzugsweise ein Bestimmen eines Demonstrationsfassungsscheibenwinkels. Außerdem umfasst das Vorhalten des zumindest einen Demonstrationsglases vorzugsweise ein Einstellen des Fassungsscheibenwinkels auf den bestimmten Demonstrationsfassungsscheibenwinkel. In einer bevorzugten Ausführungsform wird das Bestimmen eines einzustellenden Verkippungswinkels von einem Computersystem automatisch durchgeführt und insbesondere in Abhängigkeit vom Entwurf bzw. Design des zumindest einen Brillenglases vorzugsweise analytisch berechnet.

Vorzugsweise umfasst das Verfahren ein Bereitstellen einer Serie von Demonstrationsfassungen mit zumindest teilweise unterschiedlichen Fassungsscheibenwinkeln, wobei das Bestimmen eines Verkippungswinkels ein Auswählen einer Demonstrationsfassung aus der Serie von Demonstrationsfassungen in Abhängigkeit von der ermittelten Vergrößerung und/oder Verzerrung umfasst.

Damit stellt die Erfindung insbesondere einen Satz von Demonstrationsfassungen insbesondere zur Untersuchung der Verträglichkeit einer Brille mit großem Fassungsscheibenwinkel und/oder mit starker Durchmuschelung für einen Brillenträger bereit, wobei die Demonstrationsfassungen jeweils eine Gläseraufnahme zum Aufnehmen von Demonstrationsgläsern insbesondere gemäß der vorliegenden Erfindung oder einer bevorzugten Ausführungsform davon umfassen und zumindest teilweise unterschiedliche Fassungsscheibenwinkel, beispielsweise in Abstufungen von 5˚ aufweisen.

Vorzugsweise ist die Demonstrationsfassung bzw. sind die Demonstrationsfassungen zur Aufnahme austauschbarer Demonstrationsgläser ausgelegt. Demnach kann insbesondere zum Vorhalten des ausgewählten Demonstrationsglases dieses in die ausgewählte Demonstrationsfassung eingesetzt werden, welche der Brillenträger anschließend vorzugsweise zusätzlich zur gewohnten Brille während der Untersuchung trägt.

Vorzugsweise werden im Schritt des Entwerfens des Brillenglases bzw. eines Designs des Brillenglases und/oder im Schritt des Auswählens des zumindest einen afokalen Demonstrationsglases und/oder im Schritt des Bestimmens eines Verkippungswinkels individuelle Rezeptwerte, insbesondere dioptrische und/oder astigmatische Verordnungen für den Brillenträger und/oder eine individuelle Gebrauchssituation und/oder eine individuelle Gebrauchsstellung berücksichtigt. Besonders bevorzugt umfasst das Entwerfen des zumindest einen Brillenglases ein Optimieren des Brillenglases zur Korrektion einer Refraktion, insbesondere einer sphärischen und/oder astigmatischen und/oder prismatischen Verordnung, zumindest eines Auges der Brillenträgers.

Vorzugsweise umfasst das Ermitteln einer Vergrößerung und/oder Verzerrung des Brillenglases ein Ermitteln von Vergrößerungen bzw. Verzerrungen für eine Vielzahl verschiedener Blickrichtungen des Brillenträgers. Insbesondere könnten beispielsweise Vergrößerungen und/oder Verzerrungen für zwei Blickrichtungen, d.h. zwei Durchblickpunkte durch das Brillenglas ermittelt werden. Dabei wird das zumindest eine afokale Demonstrationsglas vorzugsweise derart ausgewählt und ein Verkippungswinkel so gewählt, dass die dadurch erreichte optische Wirkung für die zumindest zwei Blickrichtungen in Bezug auf die Vergrößerung und/oder Verzerrung mit den ermittelten Werten übereinstimmt oder diesen unter Berücksichtigung geeigneter Anpasskriterien (Fit-Kriterien) möglichst nahe kommt. Solche Anpasskriterien könnten beispielsweise festlegen, dass die Summe der Abweichungsbeträge oder die Summe der Quadrate der Abweichungen minimiert werden. Zur Berücksichtigung individueller Gebrauchssituationen könnten auch unterschiedliche Gewichtungen z.B. in Form von Gewichtungsfaktoren für verschiedene Blickrichtungen zur Bewertung der Anpassung vorgenommen werden. Durch eine geeignete Anpassung wird vorzugsweise zumindest dasjenige afokale Demonstrationsglas aus der Serie von bereitgestellten afokalen Demonstrationsgläser und vorzugsweise der Verkippungswinkel bestimmt, mit dem die ermittelten Werte von Vergrößerung und/oder Verzerrung am besten nachempfunden bzw. angenähert werden können.

Alternativ oder zusätzlich könnten auch Veränderungen von Vergrößerung und/oder Verzerrung bei Blickbewegung, d.h. Ableitungen der Vergrößerung bzw. Verzerrung nach dem Blickwinkel oder dem Durchblickort durch das Brillenglas ermittelt werden und als Basis für die Anpassung in Bezug auf die Auswahl eines geeigneten Demonstrationsglases und/oder die Bestimmung eines geeigneten Verkippungswinkels herangezogen werden.

Die Anpassung bzw. Optimierung, d.h. die Auswahl des zumindest einen afokalen Demonstrationsglases und/oder die Bestimmung des Verkippungswinkels wird vorzugsweise in einem bzw. mittels eines geeigneten Computersystems vorgenommen. Alternativ könnte eine Auswahl des Demonstrationsglases und/oder eine Bestimmung des Verkippungswinkels auch durch einen Optiker mittels einer Tabelle erfolgen, welche eine Vielzahl von Stützpunkten derart enthält, dass einer Vielzahl von Werten für die Vergrößerung und/oder Verzerrung die geeigneten Werte der Eigenvergrößerung des auszuwählenden afokalen Demonstrationsglases und/oder des einzustellenden Verkippungswinkels zugeordnet sind bzw. zugewiesen werden.

Vorzugsweise umfasst das Auswählen zumindest eines afokalen Demonstrationsglases ein Bereitstellen eines Demonstrationsgläsersatzes gemäß der vorliegenden Erfindung oder einer bevorzugten Ausführungsform und ein Auswählen des zumindest einen afokalen Demonstrationsglases aus diesem Demonstrationsgläsersatz.

In einem weiteren Aspekt stellt die Erfindung ein Verfahren zur Bestimmung der Verträglichkeit einer Brille mit großem Fassungsscheibenwinkel und/oder starker Durchmuschelung, insbesondere einer Sportbrille, für einen Brillenträger bereit, welches umfasst:

- Bereitstellen einer Serie von afokalen Messgläsern, d.h. zwei oder mehr afokale Messgläser, mit zumindest teilweise unterschiedlicher Eigenvergrößerung und/oder Verzerrung;
- Bestimmen zumindest eines Verträglichkeitsgrenzwerts für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers mittels der Serie von afokalen Messgläsern;
- Entwerfen zumindest eines Brillenglases bzw. eines Designs für zumindest ein Brillenglas der Brille für den Brillenträger;
- Ermitteln zumindest eines Akzeptanzbereichs des zumindest einen entworfenen Brillenglases in Abhängigkeit von dem zumindest einen bestimmten Verträglichkeitsgrenzwert.

Vorzugsweise umfasst das Bestimmen des zumindest einen Verträglichkeitsgrenzwerts mittels der Serie von afokalen Messgläsern ein Ermitteln zumindest eines Messglases aus der Serie von afokalen Messgläsern mit dem größten für den Brillenträger als noch erträglich empfundenen Wert für die Vergrößerung und/oder die Verzerrung in Bezug auf das zumindest eine Auge des Brillenträgers und/oder für den Vergrößerungsunterschied und/oder den Verzerrungsunterschied zwischen den beiden Augen des Brillenträgers.

Dieser größte Wert wird dabei vorzugsweise als der zumindest eine Verträglichkeitsgrenzwert festgelegt. Alternativ könnte aus der Serie von afokalen Messgläsern auch zumindest ein Messglas mit dem kleinsten für den Brillenträger als nicht mehr erträglich empfundenen Wert für die Vergrößerung und/oder die Verzerrung in Bezug auf das zumindest eine Auge des Brillenträgers und/oder für den Vergrößerungsunterschied und/oder den Verzerrungsunterschied zwischen den beiden Augen des Brillenträgers ermittelt werden, welcher dann als Verträglichkeitsgrenzwert festgelegt wird. Alternativ könnte der zumindest eine Verträglichkeitsgrenzwert auch in anderer Weise insbesondere in Abhängigkeit von dem größten bzw. kleinsten ermittelten Wert festgelegt werden.

Vorzugsweise umfasst das Ermitteln eines Akzeptanzbereichs:

- ein Ermitteln von Vergrößerungen und/oder Verzerrungen des zumindest einen Brillenglases und/oder von Vergrößerungsunterschieden und/oder Verzerrungsunterschieden zwischen beiden Brillengläsern der Brille für eine Vielzahl von Blickrichtungen des Brillenträgers; und

- ein Auswählen von Blickrichtungen für die die ermittelte Vergrößerung und/oder Verzerrung bzw. der ermittelte Vergrößerungs- und/oder Verzerrungsunterschied unter oder zumindest nicht über dem zumindest einen bestimmten Verträglichkeitsgrenzwert liegt.

Vorzugsweise umfasst das Ermitteln eines Akzeptanzbereichs ein Ermitteln eines Flächenbereichs des zumindest einen Brillenglases und/oder eines Blickwinkelbereichs des Brillenträgers, innerhalb dessen die Vergrößerung und/oder die Verzerrung des zumindest einen Brillenglases und/oder der Vergrößerungsunterschied und/oder der Verzerrungsunterschied zwischen beiden Brillengläsern der Brille kleiner oder zumindest nicht größer als der entsprechende bestimmte Verträglichkeitsgrenzwert ist.

Vorzugsweise werden im Schritt des Entwerfens eines Designs, d.h. im Schritt des Erstellens eines Entwurfes des Brillenglases, individuelle Rezeptwerte, insbesondere dioptrische und/oder astigmatische Verordnungen für den Brillenträger und/oder eine individuelle Gebrauchssituation und/oder eine individuelle Gebrauchsstellung berücksichtigt. Vorzugsweise wird das Entwerfen eines Designs, also das Erstellen eines Entwurfes des Brillenglases, mittels eines Computersystems nach bekannten Optimierungsalgorithmen auf Basis von individuellen Rezeptwerten, insbesondere eine dioptrische und/oder astigmatische Verordnung umfassend, durchgeführt. In einer bevorzugten Ausführungsform ist ein Computersystem ausgelegt, den zumindest einen Verträglichkeitsgrenzwert zu erfassen und den zumindest einen Akzeptanzbereich automatisch zu ermitteln. Besonders bevorzugt wird dieser anschließend graphisch, beispielsweise als Flächenbegrenzungslinie auf einer graphischen Darstellung des Brillenglases ausgegeben bzw. einem Benutzer angezeigt.

Besonders bevorzugt umfasst das Verfahren einen Schritt des vorzugsweise automatischen Bewertens des Designs (Entwurfes) beispielsweise durch Vergleichen des ermittelten Akzeptanzbereichs mit der im Hinblick auf eine insbesondere individuelle Gebrauchssituation zu erwartende Nutzung der Brille, d.h. mit dem durch das geplante Anwendungsgebiet der Brille festgelegten oder beeinflussten Blickwinkelbereich der bevorzugten oder hauptsächlichen Nutzung. Anstelle einer individuellen Gebrauchssituation könnte hier auch eine standardisierte Gebrauchssituation, z.B. für Radfahren, Golf, Skifahren, etc., einfließen.

Vorzugsweise umfasst das Bereitstellen einer Serie von afokalen Messgläsern ein Bereitstellen eines Messgläsersatzes gemäß der vorliegenden Erfindung oder einer bevorzugten Ausführungsform.

In einem weiteren Aspekt verwendet die Erfindung eine Demonstrationsfassung bzw. Demonstrationsbrillenfassung, umfassend:

- eine Fassungsschiene bzw. einen Fassungsrahmen bzw. eine Fassungsbrücke bzw. einen Fassungssteg;
- zumindest eine an der Fassungsschiene angeordnete Fixiereinrichtung zum Fixieren der Demonstrationsfassung relativ zu einer Hauptblickrichtung eines Brillenträgers; und
- zumindest ein Drehelement bzw. Montageelement zum Befestigen zumindest eines optischen Glases relativ zur Fassungsschiene derart, dass das optische Glas relativ zur Fassungsschiene um eine Verkippungsachse drehbar ist.

Als Hauptblickrichtung ist dabei insbesondere die Richtung der Fixierlinie durch den Hauptdurchblickpunkt zu verstehen. Die Verkippungsachse verläuft dabei insbesondere nicht parallel zur Hauptblickrichtung. Somit fällt die Verkippungsachse insbesondere nicht mit einer Schwenkungsachse, d.h. insbesondere einer Normalen zur Scheibenebene, und/oder der Normalenrichtung zur Fassungsebene zusammen. In einer bevorzugten Ausführungsform liegt die Verkippungsachse in der Fassungsebene der Demonstrationsfassung. Insbesondere schließt die Verkippungsachse mit der Fassungsebene vorzugsweise einen Winkel von weniger als 45˚, noch mehr bevorzugt weniger als 20˚, am meisten bevorzugt weniger als 5˚ ein, d.h. die Verkippungsachse schließt mit der Normalen zur Fassungsebene vorzugsweise einen Winkel von mehr als 45˚, noch mehr bevorzugt mehr als 70˚, am meisten bevorzugt mehr als 85˚ ein.

Insbesondere ist das zumindest eine optische Glas derart um die Verkippungsachse drehbar, dass dadurch der Fassungsscheibenwinkel der Demonstrationsfassung einstellbar ist. Vorzugsweise ist der Fassungsscheibenwinkel in einem Winkelbereich von 0˚ bis 25˚, besonders bevorzugt in einem Bereich von 0˚ bis 30˚ einstellbar.

In einem Aspekt wird insbesondere eine Demonstrationsfassung bereitgestellt, welche umfasst:

- eine Fassungsschiene bzw. einen Fassungsrahmen bzw. eine Fassungsbrücke bzw. einen Fassungssteg;
- zumindest eine an der Fassungsschiene angeordnete Fixiereinrichtung zum Fixieren der Demonstrationsfassung relativ zum Gesicht eines Brillenträgers und/oder relativ zu einer anderen Brille; und
- ein Drehelement zum Befestigen eines optischen Glases derart, dass dieses relativ zur Fassungsschiene um eine Verkippungsachse drehbar ist.

Vorzugsweise umfasst die zumindest eine Fixiereinrichtung eine Nasenauflage und/oder eine Stirnanlagefläche und/oder Bügel zum Abstützen der Demonstrationsfassung insbesondere am oder im Bereich der Ohren des Brillenträgers. In einer bevorzugten Ausführungsform umfasst die zumindest eine Fixiereinrichtung zumindest eine Fassungshalterung zum Befestigen oder Abstützen der Demonstrationsfassung an einer anderen Brille des Brillenträgers.

Vorzugsweise sind zumindest einzelne Komponenten der Fixiereinrichtung austauschbar. Insbesondere ist in einer bevorzugten Ausführungsform die Nasenauflage gegen die Fassungshalterung austauschbar. Damit ist die Demonstrationsfassung einerseits direkt auf der Nase eines Kunden oder Patienten abstützbar, insbesondere wenn dieser keine Brille trägt. Andererseits kann durch einfaches Austauschen von Teilen der Fixiereinrichtung, insbesondere durch Befestigen der Fassungshaltungen anstelle von oder zusätzlich zur Nasenauflage, die Demonstrationsfassung auch für Brillenträger eingesetzt werden, während sie eine andere Brille, z.B. ihre gewohnte Brille, tragen.

Vorzugsweise umfasst die Demonstrationsfassung außerdem eine Anzeigeeinrichtung zum Anzeigen eines eingestellten Verkippungswinkels des Drehelements.

Vorzugsweise umfasst die Demonstrationsfassung ein rechtes und ein linkes Drehelement zum Befestigen eines rechten bzw. linken optischen Glases relativ zur Fassungsschiene derart, dass die optischen Gläser relativ zur Fassungsschiene um eine rechte bzw. linke Verkippungsachse drehbar sind. Besonders bevorzugt umfasst die Demonstrationsfassung eine Betätigungseinrichtung zum synchronen Einstellen eines rechten und linken Fassungsscheibenwinkels des rechten bzw. linken optischen Glases.

In einem weiteren Aspekt schlägt die Erfindung vor, eine Demonstrationsfassung gemäß der Erfindung oder einer bevorzugten Ausführungsform davon zur Durchführung eines Verfahrens gemäß der Erfindung oder einer bevorzugten Ausführungsform davon zur insbesondere nicht-diagnostischen Untersuchung der Verträglichkeit einer Brille mit großem Fassungsscheibenwinkel und/oder mit starker Durchmuschelung, insbesondere einer Sportbrille, für einen Brillenträger zu verwenden.

In einem weiteren Aspekt bietet die Erfindung ein Computersystem zur Untersuchung der Verträglichkeit einer Brille mit großem Fassungsscheibenwinkel und/oder mit starker Durchmuschelung, insbesondere einer Sportbrille, für einen Brillenträger, umfassend:

- ein Entwurfsmodul zum Entwerfen zumindest eines Brillenglases, d.h. eines Designs für zumindest ein Brillenglas der Brille für den Brillenträger;
- eine Analyseeinrichtung zum Ermitteln einer Vergrößerung und/oder Verzerrung des zumindest einen Brillenglases für zumindest eine Blickrichtung des Brillenträgers; und
- eine Auswahleinrichtung zum Auswählen zumindest eines afokalen Demonstrationsglases mit einer bestimmten Eigenvergrößerung aus einer Serie von afokalen Demonstrationsgläsern, d.h. zwei oder mehr afokale Demonstrationsgläser, mit zumindest teilweise unterschiedlichen Eigenvergrößerungen abhängig von der ermittelten Vergrößerung und/oder Verzerrung.

Vorzugsweise umfasst das Computersystem eine Datenspeichereinrichtung zum Speichern von Identifikationsdaten einzelner Demonstrationsgläser einer Serie von afokalen Demonstrationsgläsern mit zumindest teilweise unterschiedlichen Eigenvergrößerungen. Die Auswahleinrichtung ist vorzugsweise ausgelegt zum Auswählen desjenigen afokalen Demonstrationsglases aus der Serie von afokalen Demonstrationsgläsern, das in einer Demonstrationsstellung vor dem entsprechenden Auge des Brillenträgers für die zumindest eine Blickrichtung des Brillenträgers zu einer Vergrößerung und/oder Verzerrung führt, die der von der Analyseeinrichtung ermittelten Vergrößerung bzw. Verzerrung des zumindest einen Brillenglases am nächsten kommt.

Vorzugsweise umfasst das Computersystem außerdem eine Anpasseinrichtung zum Bestimmen eines Verkippungswinkels für das zumindest eine ausgewählte afokale Demonstrationsglas abhängig von der ermittelten Vergrößerung und/oder Verzerrung des zumindest einen Brillenglases.

In einer bevorzugten Ausführungsform ist das Computersystem ausgelegt, ein Verfahren gemäß der vorliegenden Erfindung oder einer bevorzugten Ausführungsform davon zumindest teilweise auszuführen.

In einem Aspekt stellt die Erfindung ein Computersystem zur Bestimmung der Verträglichkeit einer Brille mit großem Fassungsscheibenwinkel und/oder starker Durchmuschelung, insbesondere einer Sportbrille, für einen Brillenträger bereit, welches umfasst:

- eine Erfassungseinrichtung zum Erfassen zumindest eines Verträglichkeitsgrenzwertes für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers;
- ein Entwurfsmodul zum Entwerfen zumindest eines Brillenglases, d.h. eines Designs für zumindest ein Brillenglas der Brille für den Brillenträger; und
- eine Analyseeinrichtung zum Ermitteln eines Akzeptanzbereichs des zumindest einen entworfenen Brillenglases in Abhängigkeit von dem zumindest einen erfassten Verträglichkeitsgrenzwert.

Vorzugsweise umfasst das Computersystem eine Anzeigeeinrichtung zur graphischen Darstellung eines den ermittelten Akzeptanzbereich des entworfenen Brillenglases repräsentierenden Flächen- und/oder Blickwinkelbereich. In einer bevorzugten Ausführungsform umfasst die Anzeigeeinrichtung einen Computerbildschirm, mittels dem einem Nutzer, wie z.B. einem Optiker, eine schematische Ansicht des zumindest einen Brillenglases präsentiert wird. In dieser schematischen Ansicht sind vorzugsweise der Akzeptanzbereich und/oder dessen Grenzen beispielsweise in Form von Flächen- und/oder Liniendarstellungen gekennzeichnet. Vorzugsweise ist das Computersystem außerdem ausgelegt, in der schematischen Darstellung des zumindest einen Brillenglases Bereiche häufiger Nutzung in Abhängigkeit von einer standardisierten oder individuellen Gebrauchssituation für die Brille in Form von Flächen und/oder Liniendarstellungen anzuzeigen.

Dabei ist die Erfassungseinrichtung vorzugsweise ausgelegt, eine individuelle Gebrauchssituation zu erfassen. Vorzugsweise umfasst das Computersystem außerdem eine Bewertungseinrichtung, welche ausgelegt ist, den ermittelten Akzeptanzbereich mit einem durch die Gebrauchssituation bestimmen Nutzungsbereich zu vergleichen und abhängig von einem Überlapp des Akzeptanzbereichs mit dem Nutzungsbereich eine Design-Bewertung beispielsweise in Form einer Punktezahl auszugeben, welche insbesondere ein Maß für die Verträglichkeit der Brille für den Brillenträger darstellt.

In einem Aspekt stellt die Erfindung ein computerimplementiertes Verfahren zur Untersuchung der Verträglichkeit einer Brille mit großem Fassungsscheibenwinkel und/oder mit starker Durchmuschelung für einen Brillenträger bereit, welches umfasst:

- Entwerfen zumindest eines Brillenglases, d.h. eines Designs für zumindest ein Brillenglas der Brille für den Brillenträger;
- Ermitteln einer Vergrößerung und/oder einer Verzerrung des zumindest einen Brillenglases für zumindest eine Blickrichtung des Brillenträgers; und
- Auswählen zumindest eines afokalen Demonstrationsglases mit einer bestimmten Eigenvergrößerung aus einer Serie von afokalen Demonstrationsgläsern mit zumindest teilweise unterschiedlichen Eigenvergrößerungen abhängig von der ermittelten Vergrößerung und/oder Verzerrung des zumindest einen Brillenglases.

In einem weiteren Aspekt bietet die Erfindung ein computerimplementiertes Verfahren zur Bestimmung der Verträglichkeit einer Brille mit großem Fassungsscheibenwinkel und/oder starker Durchmuschelung, insbesondere einer Sportbrille, für einen Brillenträger, welches umfasst:

- Erfassen zumindest eines Verträglichkeitsgrenzwertes für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers;
- Entwerfen zumindest eines Brillenglases, d.h. eines Designs für zumindest ein Brillenglas der Brille für den Brillenträger; und
- Ermitteln eines Akzeptanzbereichs des zumindest einen entworfenen Brillenglases in Abhängigkeit von dem zumindest einen erfassten Verträglichkeitsgrenzwert.

In einem weiteren Aspekt bietet die Erfindung ein Computerprogrammprodukt, umfassend Programmcode, der, wenn er in einem Computersystem, insbesondere in einem Computersystem gemäß der vorliegenden Erfindung oder einer bevorzugten Ausführungsform, geladen ist, dieses veranlasst, ein Verfahren gemäß der vorliegenden Erfindung oder einer bevorzugten Ausführungsform auszuführen.

In einem weiteren Aspekt stellt die Erfindung einen Demonstrationsgläsersatz, insbesondere zur Untersuchung der Verträglichkeit einer Brille mit großem Fassungsscheibenwinkel und/oder mit starker Durchmuschelung für einen Brillenträger, bereit, welcher eine Vielzahl, d.h. zwei oder mehr, von für zumindest eine spezielle Demonstrationsausgangsstellung ausgelegten Demonstrationsgläsern derart umfasst, dass die Demonstrationsgläser im wesentlichen keine dioptrische Wirkung, zumindest teilweise unterschiedliche Eigenvergrößerung und in der zumindest einen Demonstrationsausgangsstellung für zumindest eine Durchblickrichtung im wesentlichen keine Verzerrung aufweisen. Vorzugsweise umfasst der Demonstrationsgläsersatz zumindest fünf Demonstrationsgläser mit unterschiedlichen Eigenvergrößerungen.

Die Demonstrationsgläser sind insbesondere optische Gläser bzw. Linsen, insbesondere Brillengläser zur Verwendung in der Refraktionsmessbrille und/oder einer Demonstrationsfassung gemäß der vorliegenden Erfindung oder einer bevorzugten Ausführungsform, deren dioptrische Wirkung in der Demonstrationsausgangsstellung vorzugsweise zumindest in einem Bereich von mehr als 60%, noch mehr bevorzugt mehr als 75%, besonders bevorzugt mehr 90% der gesamten Glas- bzw. Linsen- bzw. Durchblickfläche und/oder des Blickwinkels durch das jeweilige Glas im Betrag kleiner als 1 dpt, vorzugsweise kleiner als 0,5 dpt, am meisten bevorzugt kleiner als 0,25 dpt ist.

In einem bevorzugten Demonstrationsgläsersatz ist die Eigenvergrößerung für jedes Demonstrationsglas über die gesamte Fläche bzw. Glasfläche im wesentlichen konstant. Insbesondere weicht für jedes Demonstrationsglas in diesem Demonstrationsgläsersatz die Eigenvergrößerung vorzugsweise zumindest in einem Bereich von mehr als 60%, noch mehr bevorzugt mehr als 75%, besonders bevorzugt mehr als 90% der gesamten Glas- bzw. Linsen- bzw. Durchblicksfläche und/oder des Blickwinkels um weniger als 2%, vorzugsweise weniger als 1%, besonders bevorzugt weniger als 0,5% von einem für jedes Demonstrationsglas spezifischen nominellen Wert bzw. Mittelwert der Eigenvergrößerung ab.

Als Demonstrationsausgangsstellung wird für den Demonstrationsgläsersatz vorzugsweise eine individuelle oder standardisierte Gebrauchsstellung bzw. - position für einen Einsatz der Demonstrationsgläser für ein Verfahren gemäß der vorliegenden Erfindung oder einer bevorzugten Ausführungsform verstanden. Dazu weist vorzugsweise jedes Demonstrationsglas eine Hauptblickrichtung auf, welche vorzugsweise im wesentlichen einem geraden, d.h. unverkippten Durchblick ohne Scheibenwinkel und/oder Vorneigung entspricht. Diese unverkippte Gebrauchsstellung stellt vorzugsweise die Demonstrationsausgangsstellung dar.

Vorzugsweise weisen die Demonstrationsgläser in der Demonstrationsausgangsstellung im wesentlichen keine Verzerrung auf. Insbesondere ist die Verzerrung der Demonstrationsgläser in der Demonstrationsausgangsstellung vorzugsweise zumindest in einem Bereich von mehr als 60%, noch mehr bevorzugt mehr als 75%, am meisten bevorzugt mehr als 90% der gesamten Glas- bzw. Linsen- bzw. Durchblickfläche und/oder des Blickwinkels nicht größer als 2,5 %, vorzugsweise nicht größer als 1 %. In einer bevorzugten Ausführungsform umfasst der Demonstrationsgläsersatz eine Vielzahl von im wesentlichen afokalen Demonstrationsgläsern, deren Verzerrung in der Demonstrationsausgangsstellung auf der gesamten Glas- bzw. Linsenfläche bzw. im gesamten Blickwinkelbereich nicht größer als 2%, noch mehr bevorzugt nicht größer als 1% ist.

Vorzugsweise liegen die zumindest teilweise unterschiedlichen Eigenvergrößerungen der Demonstrationsgläser in einem Bereich zwischen 1,01 und 1,1, besonders bevorzugt in einem Bereich zwischen 1,02 und 1,06.

Vorzugsweise weisen die Demonstrationsgläser untereinander Abstufungen ihrer Eigenvergrößerungen auf, die nicht größer als 2%, vorzugsweise nicht größer als 1% sind. So weist in einer bevorzugten Ausführungsform beispielsweise zumindest ein Demonstrationsglas des Demonstrationsgläsersatzes einen nominellen Wert bzw. Mittelwert der Eigenvergrößerung von 1,03, zumindest ein weiteres einen nominellen Wert bzw. Mittelwert von 1,04 und ein weiteres Demonstrationsglas einen nominellen Wert bzw. Mittelwert der Eigenvergrößerung von 1,05 auf.

Vorzugsweise weisen eine Vielzahl der Demonstrationsgläser eines Demonstrationsgläsersatzes gemäß der Erfindung oder einer bevorzugten Ausführungsform zumindest teilweise sphärische Vorder- und/oder Rückflächen auf. Vorzugsweise weisen die Demonstrationsgläser Basiskurven zwischen 2 dpt und 10 dpt, noch mehr bevorzugt zwischen 5 dpt und 8 dpt auf. Dabei müssen nicht alle Demonstrationsgläser dieselbe Basiskurve aufweisen. Insbesondere könnten sich Demonstrationsgläser unterschiedlicher Eigenvergrößerung in ihrer Basiskurve unterscheiden. Andererseits müssen sich nicht alle Gläser unterschiedlicher Eigenvergrößerung auch in ihrer Basiskurve unterscheiden. Insbesondere könnten sich Demonstrationsgläser mit gleicher Basiskurve beispielsweise durch eine unterschiedliche Mittendicke und/oder einen unterschiedlichen Brechungsindex in ihrer Eigenvergrößerung unterscheiden.

In einer bevorzugten Ausführungsform umfasst der Demonstrationsgläsersatz für jeden Wert der Eigenvergrößerung eine Vielzahl von Demonstrationsgläsern mit zumindest teilweise unterschiedlicher Mittendicke. Die Demonstrationsgläser gleicher Eigenvergrößerung aber unterschiedlicher Mittendicke unterscheiden sich dabei vorzugsweise in ihrer Basiskurve. Damit lassen sich viele Kombinationen verschiedener Eigenvergrößerungen mit verschiedenen Verläufen einer Verzerrung und damit eine verbesserte Genauigkeit und Flexibilität der Anpassung an die zu erwartenden optischen Eigenschaften der geplanten Brille erreichen.

Vorzugsweise weisen die Demonstrationsgläser eine Mittendicke im Bereich zwischen 2 mm und 20 mm, besonders bevorzugt zwischen 7,5 mm und 15 mm auf.

Vorzugsweise umfasst der Demonstrationsgläsersatz für jeden Wert der Eigenvergrößerung zwei Gläser, welche im wesentlichen spiegelsymmetrisch ausgestaltet sind, d.h. der Demonstrationsgläsersatz umfasst für jeden Wert der Eigenvergrößerung vorzugsweise ein linkes und ein rechtes Glas mit im wesentlichen gleicher Basiskurve und gleicher Mittendicke.

Vorzugsweise weist eine Vielzahl der Demonstrationsgläser eine torische Rückfläche derart auf, dass die astigmatische und/oder prismatische Wirkung der Demonstrationsgläser bei einer Verkippung um einen Referenzwinkel relativ zur Demonstrationsausgangsstellung zumindest in einer Hauptblickrichtung im wesentlichen verschwindet. Vorzugsweise liegt der Referenzwinkel in einem Bereich zwischen 5˚ und 25˚, weiter bevorzugt zwischen 5˚ und 20˚, noch mehr bevorzugt in einem Bereich zwischen 10˚ und 20˚, am meisten bevorzugt zwischen 10˚ und 15˚. Damit können für einen Winkelbereich, der sich für die Demonstration von optischen Effekten von Sportbrillen besonders eignet, die Abbildungseingenschaften der Demonstrationsgläser noch besser an die optischen Eigenschaften von Sportbrillen angenähert werden.

In einem weiteren bevorzugten Demonstrationsgläsersatz weist jedes der Demonstrationsgläser zumindest eine atorische Fläche auf. Vorzugsweise weist jedes Demonstrationsglas außerdem einen nasalen Referenzpunkt und einen temporalen Referenzpunkt derart auf, dass sich für jedes Demonstrationsglas der Flächenbrechwert der atorischen Fläche im nasalen Referenzpunkt von dem im temporalen Referenzpunkt unterscheidet und die Demonstrationsgläser in zumindest einem der Referenzpunkte zumindest teilweise unterschiedliche Flächenbrechwerte aufweisen. Damit lassen sich besonders einfach temporal und nasal unterschiedliche Vergrößerungen und/oder Verzerrungen realisieren. Vorzugsweise entsprechen die temporalen bzw. nasalen Referenzpunkte für alle Demonstrationsgläser dieses Demonstrationsgläsersatzes in der zumindest einen Demonstrationsausgangsstellung der gleichen temporalen bzw. nasalen Referenzblickrichtung. Vorzugsweise liegen für jedes Demonstrationsglas in der Demonstrationsausgangsstellung die Referenzpunkte, also der temporale und der nasale Referenzpunkt, in der selben horizontalen Ebene.

In einer bevorzugten Ausführungsform ist die zumindest eine atorische Fläche die Vorderfläche des jeweiligen Demonstrationsglases. Besonders bevorzugt unterscheidet dass sich für jedes Demonstrationsglas die Eigenvergrößerung im nasalen Referenzpunkt von der Eigenvergrößerung im temporalen Referenzpunkt, wobei die Demonstrationsgläser in zumindest einem der Referenzpunkte zumindest teilweise unterschiedliche Eigenvergrößerung aufweisen. Vorzugsweise liegen die zumindest teilweise unterschiedlichen Eigenvergrößerungen der Demonstrationsgläser in einem Bereich zwischen 1,0 und 1,2 besonders bevorzugt zwischen 1,01 und 1,1, noch mehr bevorzugt in einem Bereich zwischen 1,02 und 1,06. Vorzugsweise unterscheiden sich für jedes Demonstrationsglas die Eigenvergrößerungen im temporalen und nasalen Referenzpunkt um mindestens 1 %. Vorzugsweise weisen die Demonstrationsgläser untereinander Abstufungen in der Differenz ihrer Eigenvergrößerungen zwischen temporalem und nasalem Referenzpunkt auf, die nicht größer als 2%, vorzugsweise nicht größer als 1 % der Eigenvergrößerung sind.

In einer bevorzugten Ausführungsform weisen die Demonstrationsgläser untereinander Abstufungen ihrer Eigenvergrößerungen im nasalen Referenzpunkt und/oder im temporalen Referenzpunkt auf, die nicht größer als 2%, vorzugsweise nicht größer als 1% sind. So weist in einer bevorzugten Ausführungsform beispielsweise zumindest ein Demonstrationsglas des Demonstrationsgläsersatzes einen Wert der Eigenvergrößerung im nasalen oder im temporalen Referenzpunkt von 1,03, zumindest ein weiteres einen Wert von 1,04 und ein weiteres Demonstrationsglas im entsprechenden Referenzpunkt einen Wert der Eigenvergrößerung von 1,05 auf. In dem entsprechend anderen Referenzpunkt können diese Gläser insbesondere in ihrer Eigenvergrößerung übereinstimmen.

In einer weiteren bevorzugten Ausführungsform ist die zumindest eine atorische Fläche die Rückfläche des jeweiligen Demonstrationsglases. Besonders bevorzugt ist zumindest ein Fläche, vorzugsweise die Vorderfläche, als sphärische Fläche ausgebildet. Vorzugsweise weist dabei eine Vielzahl der Demonstrationsgläser dieselbe atorische Rückfläche auf, während sie sich in der Eigenvergrößerung, insbesondere in der Basiskurve der Vorderfläche und/oder der Mittendicke unterscheiden. In einer weiteren bevorzugten Ausführungsform ist die zumindest eine atorische Fläche eine sphärisch atorische Fläche.

Ein besonders bevorzugter Demonstrationsgläsersatz umfasst mehrere der hier beschriebenen bevorzugten Demonstrationsgläsersätze, d.h. der Demonstrationsgläsersatz umfasst Sätze von Demonstrationsgläsern welche sich beispielsweise in einem der genannten Parameter unterscheiden, während sie in anderen Parametern übereinstimmen. Damit lässt sich ein Raum von mehreren Parametern besonders effizient abdecken.

In einem weiteren Aspekt schlägt die Erfindung vor, einen Demonstrationsgläsersatz gemäß der Erfindung oder einer bevorzugten Ausführungsform davon vorzugsweise zusammen mit einer Demonstrationsfassung gemäß der Erfindung oder einer bevorzugten Ausführungsform davon zur Durchführung eines Verfahrens gemäß der Erfindung oder einer bevorzugten Ausführungsform davon zur insbesondere nicht-diagnostischen Untersuchung der Verträglichkeit einer Brille mit großem Fassungsscheibenwinkel und/oder mit starker Durchmuschelung, insbesondere einer Sportbrille, für einen Brillenträger zu verwenden.

In einem Aspekt stellt die Erfindung einen Messgläsersatz, insbesondere zur Bestimmung der Verträglichkeit einer Brille mit großem Fassungsscheibenwinkel und/oder starker Durchmuschelung für einen Brillenträger, bereit, welcher umfasst: eine Vielzahl von für zumindest eine Verträglichkeitsmessstellung ausgelegten Messgläsern derart, dass die Messgläser im wesentlichen keine dioptrische Wirkung, eine im wesentlichen konstante Eigenvergrößerung und in der zumindest einen Verträglichkeitsmessstellung zumindest teilweise unterschiedliche Verzerrung aufweisen. Vorzugsweise umfasst der Messgläsersatz zumindest 5 Messgläser mit unterschiedlicher Verzerrung. Vorzugsweise ist eine Vielzahl der Messgläser bitorisch, d.h. die Vorder- und die Rückfläche sind zumindest teilweise im wesentlichen torisch.

Die Messgläser sind insbesondere optische Gläser bzw. Linsen, deren dioptrische Wirkung in der Verträglichkeitsmessstellung vorzugsweise zumindest in einem Bereich von mehr als 60%, noch mehr bevorzugt mehr als 75%, besonders bevorzugt mehr 90% der gesamten Glas- bzw. Linsen- bzw. Durchblickfläche und/oder des Blickwinkels im Betrag kleiner als 1 dpt, vorzugsweise kleiner als 0,5 dpt, am meisten bevorzugt kleiner als 0,25 dpt ist. Vorzugsweise ist die Eigenvergrößerung für jedes Messglas über die gesamte Fläche bzw. Glasfläche im wesentlichen konstant. Insbesondere weicht für jedes Messglas die Eigenvergrößerung vorzugsweise zumindest in einem Bereich von mehr als 60%, noch mehr bevorzugt mehr als 75%, besonders bevorzugt mehr als 90% der gesamten Glas- bzw. Linsen- bzw. Durchblicksfläche und/oder des Blickwinkels um weniger als 2%, vorzugsweise weniger als 1%, besonders bevorzugt weniger als 0,5% von einem nominellen Wert bzw. Mittelwert der Eigenvergrößerung des Messglases ab.

Vorzugsweise ist die Verzerrung für jedes Messglas zumindest in der Verträglichkeitsmessstellung über die gesamte Fläche bzw. Glasfläche im wesentlichen konstant. Insbesondere weicht für jedes Messglas die Verzerrung vorzugsweise zumindest in einem Bereich von mehr als 60%, noch mehr bevorzugt mehr als 75%, besonders bevorzugt mehr als 90% der gesamten Glas- bzw. Linsen- bzw. Durchblicksfläche und/oder des Blickwinkels um einen Verzerrungswert von weniger als 2%, vorzugsweise weniger als 1%, besonders bevorzugt weniger als 0,5% von einem nominellen Wert bzw. Mittelwert der Verzerrung des Messglases ab.

Vorzugsweise liegen die zumindest teilweise unterschiedlichen Verzerrungen der Messgläser in einem Bereich zwischen 1 % und 20%. In einer anderen bevorzugten Ausführungsform liegen die Verzerrungen der Messgläser in einem Bereich zwischen 1 % und 10%.

Vorzugsweise weisen die Messgläser untereinander Abstufungen ihrer Verzerrungen auf, deren Verzerrungswert nicht größer als 2%, vorzugsweise nicht größer als 1% sind, d.h. der Unterschied bzw. die Differenz der Verzerrungen in der Abstufung von Verzerrungen ist vorzugsweise nicht größer als 2%, noch mehr bevorzugt nicht größer als 1%. So umfasst beispielsweise ein bevorzugter Messgläsersatz jeweils zumindest ein Messglas mit einem Verzerrungswert von 1%, 2%, 3%, etc..

Vorzugsweise umfasst der Messgläsersatz für jeden Wert der Verzerrung zwei Gläser umfasst, welche im wesentlichen spiegelsymmetrisch ausgestaltet sind und/oder mit einer um 90˚ verdrehten Orientierung der Verzerrung einem linken bzw. rechten Auge vorhaltbar sind. Damit kann mit einem relativ niedrigen Wert einer gesamten Verzerrung bereits durch Vorhalten der beiden Gläser mit einer um 90˚ verdrehten Orientierung der Verzerrung ein großer Verzerrungsunterschied zwischen den beiden Augen des Brillenträgers getestet bzw. untersucht werden.

In einem weiteren Aspekt schlägt die Erfindung vor, einen Messgläsersatz gemäß der Erfindung oder einer bevorzugten Ausführungsform davon zur Durchführung eines Verfahrens gemäß der Erfindung oder einer bevorzugten Ausführungsform davon zur Bestimmung der Verträglichkeit einer Brille mit großem Fassungsscheibenwinkel und/oder mit starker Durchmuschelung, insbesondere einer Sportbrille, für einen Brillenträger zu verwenden.

Unter einem Entwurf oder "Design" eines Brillenglases wird im Sinne der Erfindung insbesondere Form und/oder Position bzw. Verlauf von Vorderfläche und Rückfläche des Brillenglases sowie das Material des Glases insbesondere zur Erreichung einer auf Rezeptanforderungen des Brillenträger abzielende optischen Wirkung, d.h. die optischen Eigenschaften des Glases insbesondere im Hinblick auf beispielsweise eine dioptrische und/oder astigmatische Wirkung für eine spezifische an den Brillenträger angepasste Gebrauchsstellung und evtl. die vom Brillenträger beabsichtigte Gebrauchssituation verstanden. Durch die beabsichtigte Gebrauchssituation könnten insbesondere die beabsichtigten Anwendungsbedingungen der Brille und eine damit verbundene unterschiedliche Gewichtung einzelner Flächen- bzw. Blickrichtungsbereiche beim Entwerfen bzw. Optimieren des Designs ebenso wie bei einer Auswahl der zumindest einen Blickrichtung für die Ermittlung der Vergrößerung und/oder Verzerrung sowie bei der Auswahl des Demonstrationsglases bzw. der Bestimmung des Verkippungswinkels berücksichtigt werden. Das Entwerfen eines Designs erfolgt vorzugsweise über bekannte Optimierungsalgorithmen mittels eines Computersystems auf Basis der zuvor festgelegten individuellen Parameter, wie z.B. die Rezeptanforderungen.

Vorzugsweise wird die Verzerrung des Brillenglases bei zumindest einer Blickrichtung des Brillenträgers für eine individuelle Gebrauchsstellung ermittelt. In einer anderen bevorzugten Ausführungsform könnte die Verzerrung auch für eine standardisierte Gebrauchsstellung ermittelt werden. Insbesondere könnten für eine standardisierte Gebrauchsstellung und für eine Vielzahl von Brillengläsern mit zumindest teilweise unterschiedlichen optischen Eigenschaften die zu erwartenden Vergrößerungen und/oder Verzerrungen in einer Datenbank bereitgestellt werden. Die tatsächlichen Werte der Verzerrung für ein individuelles Design bei der standardisierten oder einer individuellen Gebrauchssituation könnten dann durch Interpolation bzw. Extrapolation aus den vorbestimmten bzw. bereitgestellten Werten ermittelt werden. In einer anderen bevorzugten Ausführungsform werden für eine Vielzahl von Brillengläsern mit zumindest teilweise unterschiedlichen optischen Eigenschaften die zu erwartenden Vergrößerungen und/oder Verzerrungen bei zumindest einer standardisierten Gebrauchssituation in einer Tabelle bereitgestellt, welche von einem Optiker zum Ermitteln bzw. Abschätzen der im individuellen Fall zu erwartenden Vergrößerungen und/oder Verzerrungen herangezogen werden kann.

Insbesondere könnte eine Gebrauchsstellung bzw. Gebrauchsposition beispielsweise anhand einer standardisierten Gebrauchsstellung bzw. Gebrauchsposition festgelegt werden. Bei Verwendung einer Brille bzw. Brillenfassung gemäß einer bevorzugten, standardisierten Gebrauchsstellung beträgt der Augendrehpunktabstand etwa 27,4 mm oder etwa 27,9 mm oder etwa 28,5 mm oder etwa 28,8 mm, die Vorneigung, d.h. der pantoskopische Winkel beträgt etwa 8˚, der Fassungsscheibenwinkel beträgt etwa 0˚, die Pupillendistanz beträgt etwa 63 mm, der Hornhautscheitelabstand beträgt etwa 15 mm, die Objektentfernung im Fernbezugspunkt beträgt etwa 0 dpt und die Objektentfernung im Nahbezugspunkt beträgt etwa -2,5 dpt.

Insbesondere beträgt bei einer Verwendung einer Brille bzw. Brillenfassung gemäß einer weiteren standardisierten Gebrauchsstellung der Augendrehpunktabstand etwa 26,5 mm, die Vorneigung, d.h. der pantoskopische Winkel etwa 9˚, der Fassungsscheibenwinkel etwa 5˚, die Pupillendistanz etwa 64 mm und der Hornhautscheitelabstand beträgt etwa 13 mm.

Alternativ beträgt bei einer Verwendung einer Brille bzw. Brillenfassung gemäß einer standardisierten Gebrauchsstellung der Augendrehpunktabstand etwa 28,5 mm, die Vorneigung, d.h. der pantoskopische Winkel etwa 7˚, der Fassungsscheibenwinkel etwa 0˚, die Pupillendistanz etwa 63 mm und der Hornhautscheitelabstand beträgt etwa 15 mm.

Alternativ beträgt bei einer Verwendung einer Brille bzw. Brillenfassung gemäß einer standardisierten Gebrauchsstellung der Augendrehpunktabstand etwa 25 mm, die Vorneigung, d.h. der pantoskopische Winkel etwa 8˚, der Fassungsscheibenwinkel etwa 5˚, die Pupillendistanz etwa 64 mm und der Hornhautscheitelabstand beträgt etwa 13 mm.

Alternativ beträgt bei einer Verwendung einer Brille bzw. Brillenfassung gemäß einer standardisierten Gebrauchsstellung der Augendrehpunktabstand etwa 27,5 mm, die Vorneigung, d.h. der pantoskopische Winkel etwa 11˚, der Fassungsscheibenwinkel etwa 0˚, die Pupillendistanz etwa 65 mm und der Hornhautscheitelabstand beträgt etwa 14 mm.

Beispielsweise beträgt bei einer Verwendung einer Brille bzw. Brillenfassung gemäß einer standardisierten Gebrauchsstellung der Augendrehpunktabstand etwa 28,5 mm und der Hornhautscheitelabstand beträgt etwa 15 mm.

Die Fassungsebene im Sinne dieser Beschreibung entspricht im wesentlichen der Fassungsebene, wie sie in einschlägigen Normen wie beispielsweise der DIN EN ISO 8624, der DIN 58208, DIN EN ISO 13666, der DIN 5340 und der DIN EN ISO 8980-2 definiert ist. Ferner wird bei den verwendeten Fachbegriffen zusätzlich zu den genannten Normen weiterhin auf Definitionen in dem Fachbuch "Die Optik des Auges und der Sehhilfen" von Dr. Roland Enders, 1995 Optische Fachveröffentlichung GmbH, Heidelberg, sowie in dem Fachbuch "Optik und Technik der Brille" von Heinz Diepes und Ralf Blendowske, 2002 Verlag Optische Fachveröffentlichungen GmbH, Heidelberg, verwiesen. Die Normen sowie die genannten Bücher stellen für die Begriffsdefinitionen insoweit einen integralen Offenbarungsbestandteil der vorliegenden Beschreibung der Erfindung dar. Die Fassungsebene entspricht insbesondere der Fassungsebene in dem herkömmlichen Kastensystem, welches ebenfalls in den genannten Normen definiert ist.

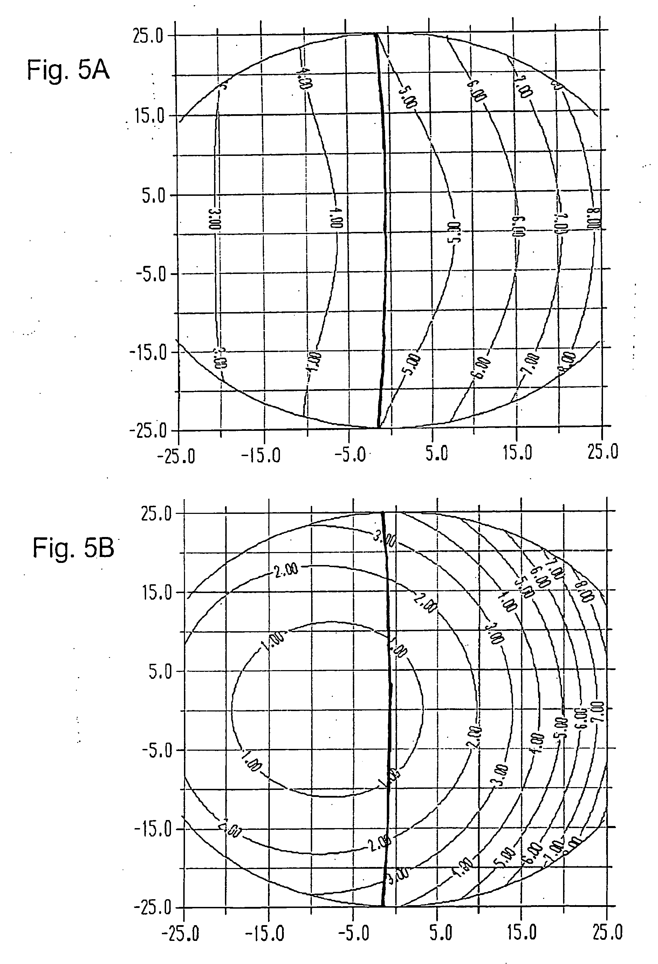

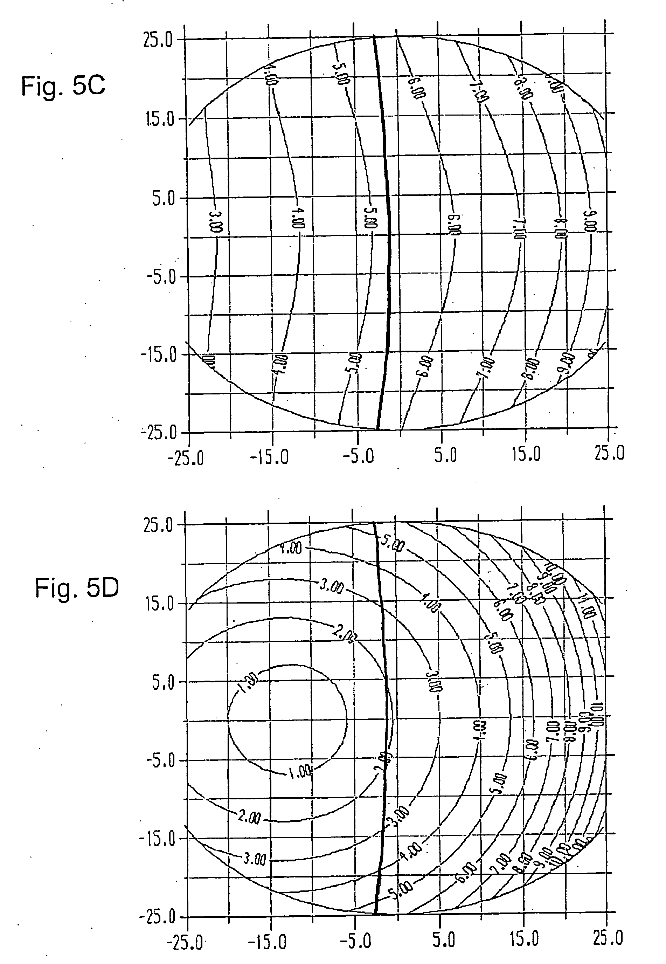

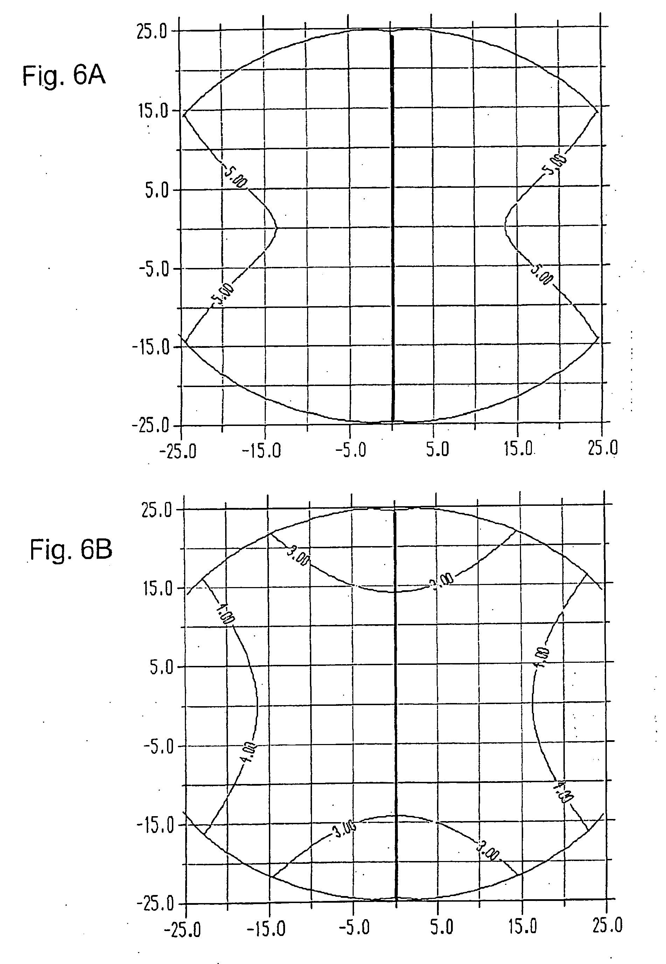

Unter einer Bemaßung im Kastenmaß wird im Sinne dieser Beschreibung das Maßsystem verstanden, wie es in einschlägigen Normen, beispielsweise in der DIN EN ISO 8624 und/oder der DIN EN ISO 1366 DIN und/oder der DIN 58 208 und/oder der DIN 5340, beschrieben wird. Ferner wird hinsichtlich des Kastenmaßes und weiterer verwendeter herkömmlicher Begriffe und Parameter auf das Buch "Die Optik des Auges und der Sehhilfen" von Dr. Roland Enders, 1995 Optische Fachveröffentlichung GmbH, Heidelberg, sowie das Buch "Optik und Technik der Brille" von Heinz Diepes und Ralf Blendowske, 2002 Verlag Optische Fachveröffentlichungen GmbH, Heidelberg, verwiesen. Ebenso wird auch auf die Broschüre "inform fachberatung für die augenoptik" PR-Schriftenreihe des ZVA für den Augenoptiker, Heft 9, "Brillenzentrierung", ISBN 3-922269-23-0, 1998 verwiesen, in welcher das Kastenmaß insbesondere in Figuren 5 und 6 beispielhaft dargestellt ist. Weiterhin wird auch auf das Buch "Brillenanpassung Ein Schulbuch und Leitfaden" von Wolfgang Schulz und Johannes Eber 1997, DOZ-Verlag, herausgegeben vom Zentralverband der Augenoptiker, Düsseldorf, ISBN 3-922269-21-4 verwiesen, insbesondere auf Punkte 1.3, 1.4. und 1.5 und die zugehörigen Abbildungen. Die Normen, die genannte Broschüre sowie die genannten Bücher stellen für die Begriffsdefinitionen insoweit einen integralen Offenbarungsbestandteil der vorliegenden Anmeldung dar.

Die Begrenzung nach einer Bemaßung im Kastenmaß umfasst beispielsweise Fassungspunkte für ein Auge oder beide Augen, welche am weitesten außen bzw. innen und/oder oben bzw. unten liegen. Diese Fassungspunkte werden herkömmlicherweise anhand von Tangenten an die Brillenfassung bzw. den jeweiligen Augen zugeordneten Bereichen der Brillenfassung bestimmt (vgl. DIN 58 208; Bild 3).

Insbesondere ist das Kastenmaß ein ein Brillenglas umschreibendes Rechteck in der Scheibenebene. Gemäß oben genannter Normen wird zur Bestimmung der Scheibenebene mathematisch von einer Ebene mit dem Normalenvektor des Kreuzprodukts von Mittelparallele/-horizontale des Kastens ausgegangen. Beispielsweise lässt sich die Normale der Scheibenebene als Senkrechte zu zwei Geraden bestimmen, von denen die eine durch den nasalen Punkt und den temporalen Punkt und die andere durch den oberen und den unteren Punkt des Glasrandes zur Fassung bestimmt ist. Liegt der Scheibenrand in einer Ebene, wo kreuzen sich die beiden Geraden, liegen also ebenfalls in einer Ebene. Liegen die beiden Geraden nicht in einer Ebene, liegt insbesondere die Gerade durch den nasalen Punkt und den temporalen Punkt näher am Auge eines Brillenträgers als die Gerade durch den oberen und den unteren Punkt des Glasrandes, wie dies insbesondere bei Sportbrillen oft auftritt, so spricht man beispielsweise von Durchmuschelung. Die Durchmuschelung bezeichnet also insbesondere eine Abweichung eines Scheibenrandes von einer Ebene. Als Maß für die Durchmuschelung könnte insbesondere der gegenseitige Abstand der beiden genannten Geraden herangezogen werden. Vorteilhafterweise entsprechen hier die Vorneigung und der Fassungsscheibenwinkel am besten der Durchblicksituation.

Der "Haltepunkt" für die Scheibenebene wird folgendermaßen genähert: Ausgangspunkt ist die Mitte der Strecke zwischen dem oberen und dem unteren Punkt. Anschließend wird horizontal entlang dem Vektor zwischen nasalem Punkt und temporalen Punkt in der Mitte der Scheibe (genähert durch die x-Koordinate) gefolgt. Das Kreuzprodukt aus dem Vektor zwischen den Mitten der Scheibenebenen beider Seiten und dem Mittelwert der beiden Vektoren aus oberem und unterem Fassungspunkt bestimmt die Normale der Fassungsebene. Haltepunkt ist eine der Scheibenmitten.

Das Kastenmaß wird als senkrechte Projektion des Scheibenrandes auf die Scheibenebene bestimmt. Der Fassungsscheibenwinkel kann nun sogar für jede Seite als der Winkel zwischen der jeweiligen Scheibenebene und der Fassungsebene bestimmt werden.

In anderen Worten läßt sich die Normale der Scheibenebene aus dem Kreuzprodukt des Vektors zwischen dem nasalen und dem temporalen Schnittpunkt einer horizontalen Ebene durch die Gerade der Nullblickrichtung mit dem jeweiligen Glasrand zur Fassung sowie dem Vektor zwischen dem oberen und dem unteren Schnittpunkt einer vertikalen Ebene durch die Gerade Nullblickrichtung mit den jeweiligen Glasrand zur Fassung bestimmen.

Die Erfindung wird nachfolgend mit Bezug auf begleitende Zeichnungen bevorzugter Ausführungsformen beispielhaft beschrieben. Dabei zeigen:

Fig. 1: eine schematische Darstellung eines beispielhaften Koordinatensystems zur Beschreibung der Anordnung und Verkippung eines Brillenglases bzw. Demonstrationsglases in einer Gebrauchsstellung relativ zum Auge;

Fig. 2A - 2D: berechnete Verteilungen der Vergrößerung (Fig. 2A und Fig. 2C) bzw. der Verzerrung (Fig. 2B und Fig. 2D) für ein erstes beispielhaftes Brillenglases bei einem Verkippungswinkel von 15˚ (Fig. 2A und Fig. 2B) bzw. 20˚ (Fig. 2C und Fig. 2D);

Fig. 3A - 3D: berechnete Verteilungen der Vergrößerung (Fig. 3A und Fig. 3C) bzw. der Verzerrung (Fig. 3B und Fig. 3D) für ein zweites beispielhaftes Brillenglases bei einem Verkippungswinkel von 15˚ (Fig. 3A und Fig. 3B) bzw. 20˚ (Fig. 3C und Fig. 3D);

Fig. 4A - 4F: berechnete Verteilungen der Vergrößerung (Fig. 4A, Fig. 4C und Fig. 4E) bzw. der Verzerrung (Fig. 4B, Fig. 4D und Fig. 4F) für ein afokales Demonstrationsglas gemäß einer bevorzugten Ausführungsform der Erfindung bei Verkippungswinkeln von 0˚ (Fig. 4A und Fig. 4B), 15˚ (Fig. 4C und Fig. 4D) bzw. 25˚ (Fig. 4E und Fig. 4F);

Fig. 5A - 5D: berechnete Verteilungen der Vergrößerung (Fig. 5A und Fig. 5C) bzw. der Verzerrung (Fig. 5B und Fig. 5D) für ein afokales Demonstrationsglas gemäß einer weiteren bevorzugten Ausführungsform der Erfindung bei Verkippungswinkeln von 15˚ (Fig. 5A und Fig. 5B) bzw. 25˚ (Fig. 5C und Fig. 5D);

Fig. 6A - 6B: berechnete Verteilungen der Vergrößerung (Fig. 6A) bzw. der Verzerrung (Fig. 6B) für ein afokales Messglas gemäß einer bevorzugten Ausführungsform der Erfindung; und

Fig. 7: eine schematische Darstellung einer Demonstrationsfassung mit einem eingesetzten Demonstrationsmessglas gemäß einer bevorzugten Ausführungsform der Erfindung.

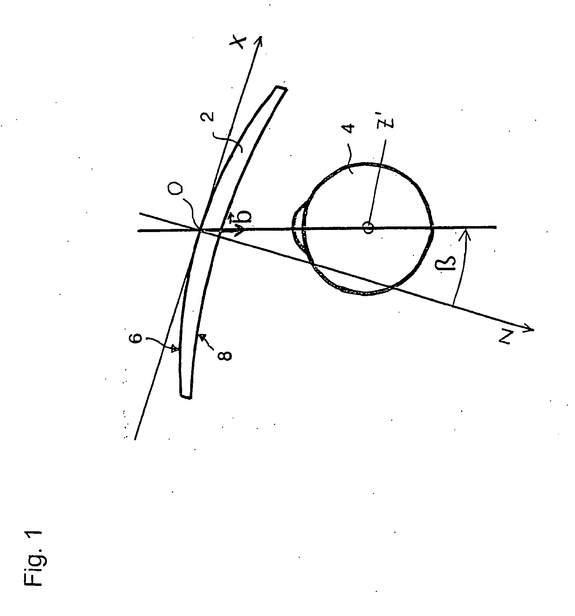

Fig. 1 zeigt eine schematische Darstellung einer beispielhaften Positionierung eines Demonstrationsglases 2 relativ zu einem Auge 4 eines Brillenträgers mit einem Augendrehpunkt Z'. Der Koordinatenursprung O eines x-y-z-Koordinatensystems liegt in der dargestellten bevorzugten Ausführungsform auf der Vorderfläche 6 des Glases, insbesondere des Demonstrationsglases 2. In einer bevorzugten Ausführungsform liegt der Koordinatenursprung O im Scheitelpunkt der Vorderfläche 6. In einer anderen bevorzugten Ausführungsform liegt der Koordinatenursprung im geometrischen Mittelpunkt oder im Schwerpunkt der Vorderfläche 6. Die x-y-Ebene des Koordinatensystems verläuft vorzugsweise tangential zur Vorderfläche 6, wobei in einer bevorzugten Anordnung des Glases, insbesondere des Demonstrationsglases 2 vor dem Auge 4 des Brillenträgers die x-Richtung in einer horizontalen Ebene und y-Richtung in einer vertikalen Ebene verläuft. Die z-Richtung zeigt vorzugsweise zum Auge hin, d.h. zu dem Halbraum, in dem das Auge 4 liegt, also zum Glas hin. In einer bevorzugten Ausführungsform, insbesondere eines nichtprismatischen Glases fällt die z-Achse mit der optischen Achse des Glases zusammen und verläuft durch den Scheitelpunkt der Rückfläche 8 des Glases.

Wie in Fig. 1 dargestellt, erfolgt die Verkippung des Glases 2 ohne Vorneigung ($\alpha = 0$) vorzugsweise um eine zur y-Achse parallele Achse, am meisten bevorzugt um die y-Achse. Ohne Vorneigung ($\alpha = 0$) gilt als Verkippungswinkel $\beta$ vorzugsweise der Winkel zwischen der z-Achse und einem Vektor $\vec{b}$ vom Koordinatenursprung O in Richtung zum Augendrehpunkt Z', also der Winkel zwischen z-Achse und einer durch den Koordinatenursprung und den Augendrehpunkt Z' verlaufenden Gerade. Andererseits erfolgt die Vorneigung des Glases 2 ohne Verkippung ($\beta = 0$) vorzugsweise um eine zur x-Achse parallele Achse, am meisten bevorzugt um die x-Achse. Ohne Verkippung ($\beta = 0$) gilt als Vorneigung bzw. Vorneigungswinkel $\alpha$ vorzugsweise der Winkel zwischen der z-Achse und dem Vektor $\vec{b}$ vom Koordinatenursprung O in Richtung zum Augendrehpunkt Z', also der Winkel zwischen z-Achse und der durch den Koordinatenursprung und den Augendrehpunkt Z' verlaufenden Gerade. Bei Nullblickrichtung verläuft der Sehstrahl für ein nichtprismatisches Glas ohne Verkippung ($\beta = 0$) und ohne Vorneigung ($\alpha = 0$) vorzugsweise entlang der z-Achse. Im allgemeinen Fall gilt unter Berücksichtigung der Vorneigung $\alpha$ und der Verkippung $\beta$ im x-y-z-Koordinatensystem vorzugsweise

$$\vec{b} = \begin{pmatrix} b_x \\ b_y \\ b_z \end{pmatrix} = \frac{1}{\sqrt{1 - \sin^2\alpha \cdot \sin^2\beta}} \begin{pmatrix} \cos\alpha \cdot \sin\beta \\ -\sin\alpha \cdot \cos\beta \\ \cos\alpha \cdot \cos\beta \end{pmatrix}$$

Vorzugsweise bildet eine Stellung des Demonstrationsglases 2 vor dem Auge 4 ohne Vorneigung $\alpha = 0$ und ohne Verkippung $\beta = 0$ eine Demonstrationsausgangsstellung.

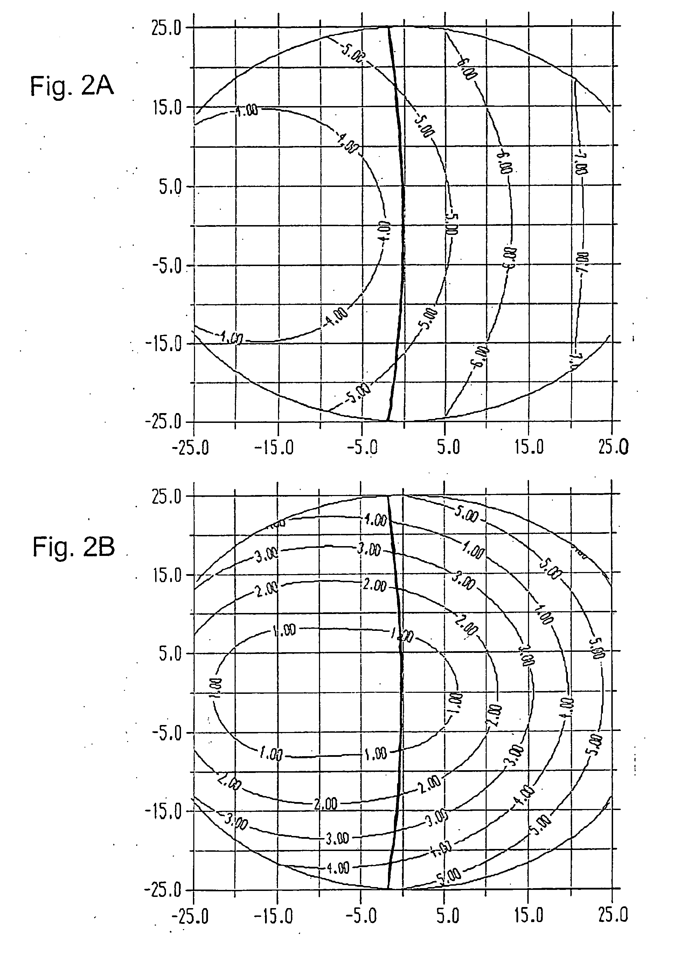

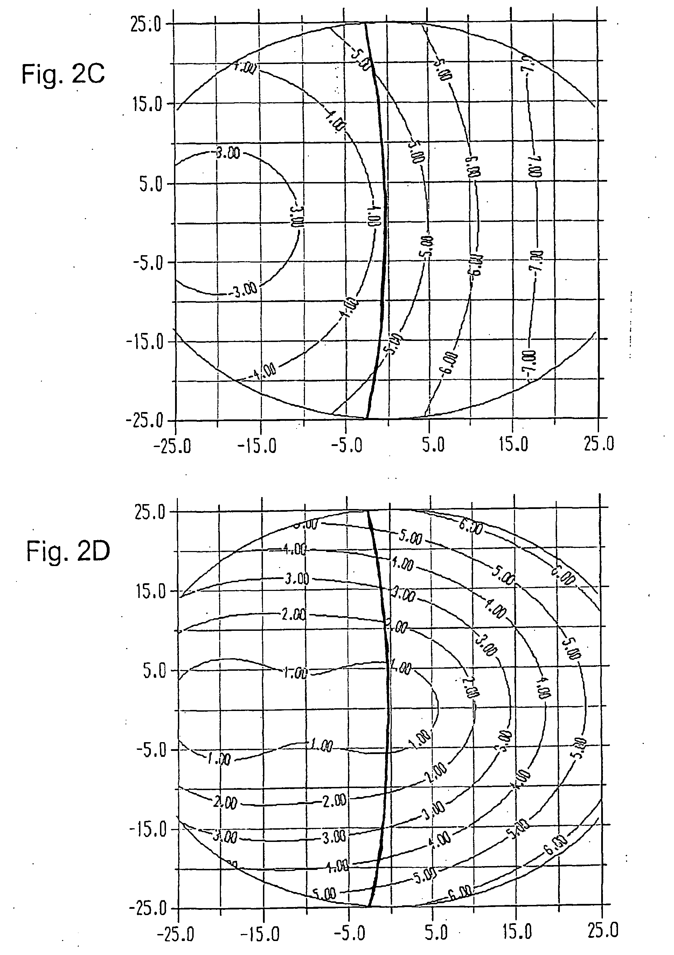

Fig. 2 zeigt die Verteilungen bzw. den Verlauf von Vergrößerung und Verzerrung in Abhängigkeit von der Durchblickstelle durch die Vorderfläche für ein erstes beispielhaftes Brillenglas einer Sportbrille vom Typ "SingleVision Sport Perfalit 1.5" mit einer Basiskurve von 7,0 dpt und einer sphärischen Wirkung von -3,0 dpt. Für diese und die nachfolgenden Figuren weist x > 0 zum nasalen Bereich und x < 0 zum temporalen Bereich des Brillenglases. Der Koordinatenursprung des dargestellten kartesischen Koordinatensystems fällt jeweils mit dem Scheitelpunkt der Vorderfläche des jeweiligen Glases zusammen und die z-Achse bildet die optische Achse des jeweiligen Glases und ist zum Auge hin orientiert. Die x-Achse eine horizontale Achse in der jeweiligen Gebrauchsstellung fest. Die angegebenen Zahlenwerte des Koordinatensystems entsprechen den x- bzw. y-Positionen der Durchstoßpunkte der Hauptstrahlen durch die Vorderfläche in Millimetern. Die dick gezeichnete, annähernd vertikale Linie nahe der y-Achse in den nachfolgenden Figuren stellt jeweils die Hauptblicklinie beim Blick nach unendlich dar. Die Verkippung erfolgt durch eine Drehung um die y-Achse. Die Werte für die in den nachfolgenden Figuren dargestellten optischen Eigenschaften wurden für einen Hornhautscheitelabstand (HSA) von 15 mm und einen Augendrehpunktabstand von 28,5 mm angegeben. Beispielsweise bestimmen diese Parameter zumindest teilweise eine Demonstrationsausgangsstellung eines bevorzugten Demonstrationsgläsersatzes.

Fig. 2A zeigt die Verteilung bzw. den Verlauf der Vergrößerung des Brillenglases in Abhängigkeit von der Durchblickstelle durch die Vorderfläche bei einem Verkippungswinkel des Brillenglases von 15˚. Die dargestellten dünnen, gekrümmten Linien bezeichnen Linien konstanter Vergrößerung in einer Abstufung von 1%. Von links nach rechts repräsentieren die Linien somit jeweils Vergrößerungen von 1,04; 1,05; 1,06 bzw. 1,07. Wie in Fig. 2A dargestellt, nimmt die Vergrößerung des gezeigten Brillenglases vom temporalen zum nasalen Bereich von einem Wert zwischen 1,03 und 1,04 auf einen Wert über 1,07 kontinuierlich zu. Diese asymmetrische Verteilung der Vergrößerung zwischen temporalem und nasalem Bereich des Brillenglases ist eine Konsequenz der Verkippung des Brillenglases um eine im wesentlichen zur y-Richtung, d.h. vertikaler Richtung, parallelen Achse.

Fig. 2B zeigt die Verteilung bzw. den Verlauf der Verzerrung für das Brillenglas von Fig. 2A bei demselben Verkippungswinkel von 15˚. Wie die Vergrößerung steigt auch die Verzerrung zum nasalen Bereich des Brillenglases hin an. Die dargestellten Linien konstanter Verzerrung repräsentieren jeweils Verzerrungen von 1% (innerste Linie) bis 6% in Schritten von 1%. Während die Verzerrung im zentralen Bereich mit unter 1 % vergleichsweise klein ist, steigt sie zu den peripheren Bereichen des Brillenglases und insbesondere zum nasalen Bereich hin deutlich an.

Fig. 2C zeigt die Verteilung bzw. den Verlauf der Vergrößerung für das Brillenglas von Fig. 2A bei einem Verkippungswinkel von 20˚. Die eingezeichneten Linien konstanter Vergrößerung repräsentieren Vergrößerungswerte von 1,03 bis 1,07 in Schritten von 1%. Wie für den Fall der in Fig. 2A gezeigten Verkippung um 15˚ nimmt auch bei der in Fig. 2C gezeigten Verkippung um 20˚ die durch das Brillenglas bewirkte Vergrößerung zum nasalen Bereich hin zu. Dabei nimmt mit steigendem Verkippungswinkel der Unterschied in der Vergrößerung zwischen nasalem und temporalem Bereich des Brillenglases zu.

Fig. 2D zeigt die Verteilung bzw. den Verlauf der Verzerrung für das Brillenglas von Fig. 2C bei demselben Verkippungswinkel von 20˚. Die dargestellten Linien konstanter Verzerrung repräsentieren nacheinander jeweils Verzerrungswerte von 1% (innerste Linie) bis 6% in Schritten von 1 %. Wie für den Fall der in Fig. 2B gezeigten Verkippung um 15˚ nimmt auch bei der in Fig. 2D gezeigten Verkippung um 20˚ die durch das Brillenglas bewirkte Verzerrung zu den peripheren Bereichen des Brillenglases und insbesondere zum nasalen Bereich hin zu. Dabei nimmt mit steigendem Verkippungswinkel der Unterschied in der Verzerrung zwischen nasalem und temporalem Bereich des Brillenglases zu.

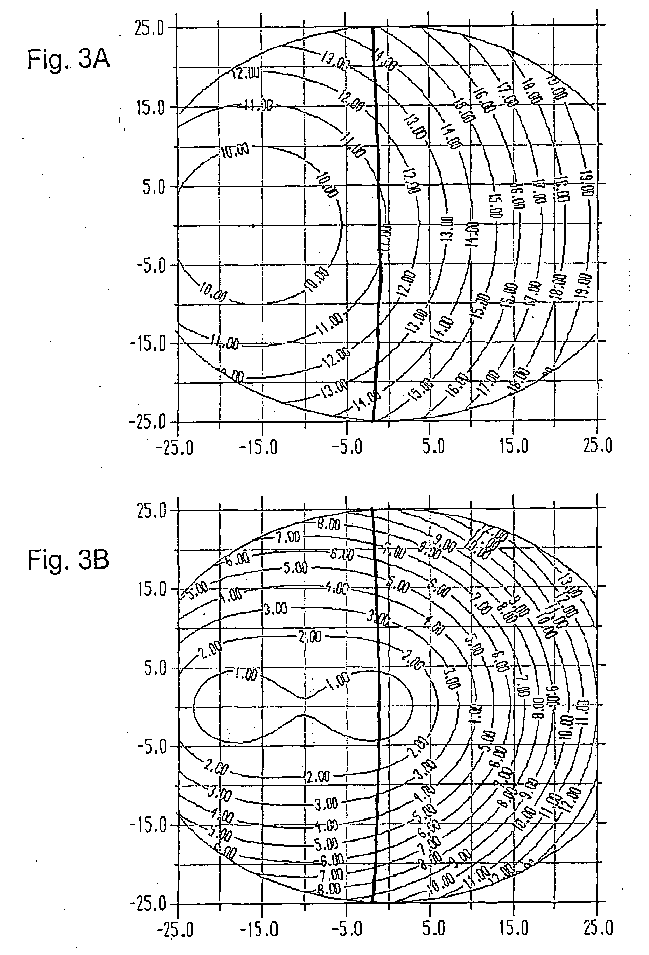

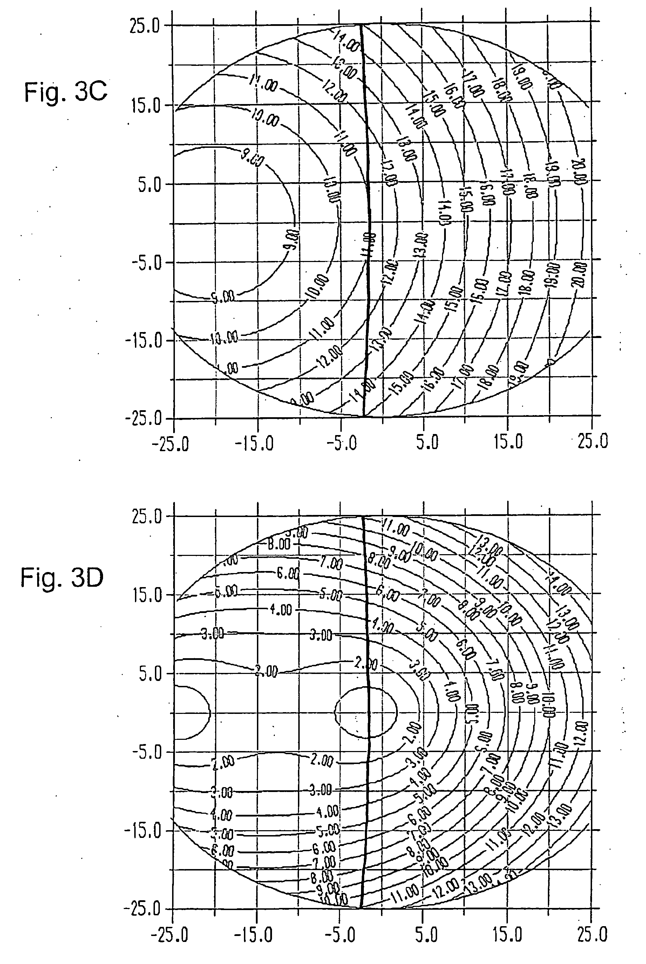

**Fig. 3** zeigt die Verteilung bzw. den Verlauf von Vergrößerung und Verzerrung in Abhängigkeit von der Durchblickstelle durch die Vorderfläche für ein zweites beispielhaftes Brillenglas einer Sportbrille vom Typ "SingleVision Sport P15" mit einer Basiskurve von 7,0 dpt und einer sphärischen Wirkung von +3,0 dpt. Mit denselben Konventionen für das Koordinatensystem wie in Fig. 2 sind in Fig. 3 die Werte für die Vergrößerung und die Verzerrung in analoger Weise ebenfalls für Verkippungswinkel von 15˚ bzw. 20˚ dargestellt.

Insbesondere zeigt Fig. 3A die Verteilung bzw. den Verlauf der Vergrößerung für das zweite beispielhafte Brillenglas bei einem Verkippungswinkel des Brillenglases von 15˚. Die dargestellten Linien konstanter Vergrößerung repräsentieren nacheinander Werte für die Vergrößerung von 1,10 (innerste Linie) bis 1,19 in Schritten der Vergrößerungswerte von 1%. Der sich unter denselben Bedingungen ergebende Verlauf der Verzerrung ist in Fig. 3B gezeigt, wobei die dargestellten Linien konstanter Verzerrung nacheinander jeweils Werte der Verzerrung von 1 % (innerste Linie) bis 14% in Schritten von 1 % repräsentieren.

Fig. 3C und Fig. 3D zeigen die Verteilung der Vergrößerung bzw. der Verzerrung für das Brillenglas von Fig. 3A und Fig. 3B bei einem Verkippungswinkel von 20˚. Dabei repräsentieren die in Fig. 3C dargestellten Linien konstanter Vergrößerung jeweils Werte der Vergrößerung von 1,09 (innerste Linie in der linken Hälfte) bis 1,20 in Schritten der Vergrößerungswerte von 1%. Die Linien konstanter Verzerrung in Fig. 3D repräsentieren jeweils Werte für die Verzerrung von 1 % (innerste Linie) bis 14% in Schritten von 1%.

Wegen der höheren Durchbiegung (z.B. Basiskurven im Bereich von 6 bis 8 dpt). und der oft größeren Mittendicke, welche unter anderem aus den großen Scheibenlängen von Sportbrillen resultiert, haben Gläser für Sportbrillen meist schon unverkippt und zentral eine stärkere Vergrößerung als normale Korrektionsgläser. Darüber hinaus ändern sich durch die starke Verkippung der Brillengläser in vielen Sportbrillen die Abbildungseigenschaften zusätzlich. Auswirkungen der Verkippung sind beispielsweise aus Fig. 2 und Fig. 3 ersichtlich. So ergeben sich beispielsweise starke nasale und temporale Unterschiede in der Vergrößerung und/oder Verzerrung, d.h. in korrespondierenden Durchblickstellen von rechtem und linkem Auge sind insbesondere bei seitlichen Blickwinkeln relativ große Differenzen der Vergrößerung bzw. Verzerrung zu erwarten. Die Vergrößerung ist generell und die Verzerrung ist insbesondere in den peripheren Bereichen höher als bei normalen Korrektionsgläsern. Die Gradienten der Vergrößerung und/oder Verzerrung sind insbesondere im nasalen Bereich relativ hoch.

Mittels der vorliegenden Erfindung können solche optischen Eigenschaften von insbesondere individuellen Sportbrillen in einfacher Weise simuliert und einem Brillenträger erfahrbar gemacht werden bevor er die eigentliche Sportbrille bestellt. Dazu wird insbesondere ein Demonstrationsgläsersatz mit einer Vielzahl von für zumindest eine spezielle Demonstrationsausgangsstellung ausgelegten Demonstrationsgläsern bereitgestellt.

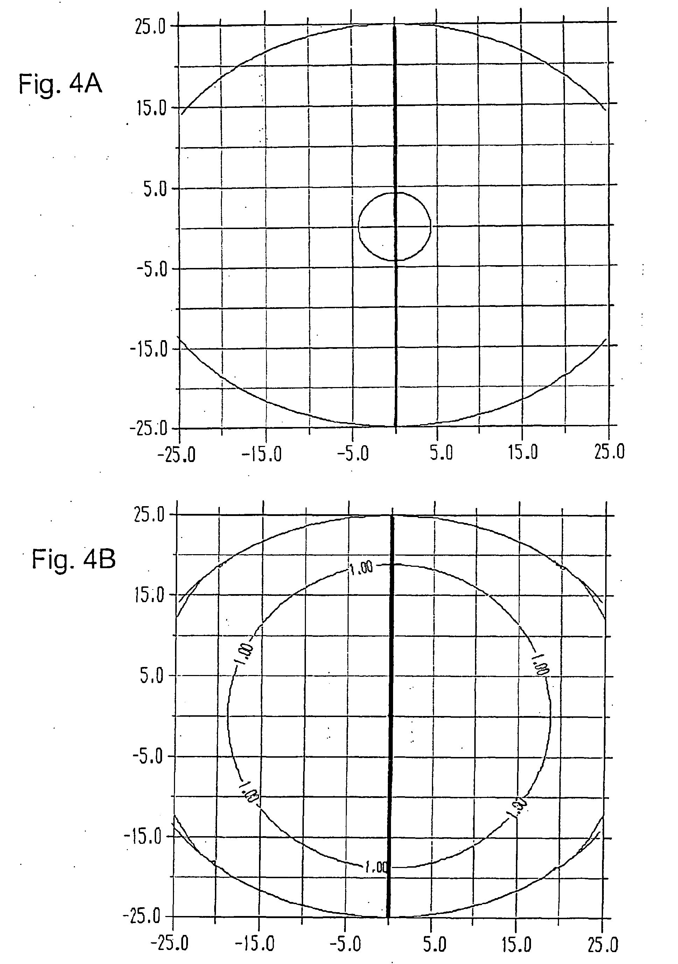

**Fig. 4A** bis **Fig. 4F** zeigen berechnete Vergrößerungen bzw. Verzerrungen in Abhängigkeit von der Durchblickstelle durch die Vorderfläche für ein afokales Demonstrationsglas gemäß einer bevorzugten Ausführungsform eines Demonstrationsgläsersatzes der Erfindung bei verschiedenen Verkippungswinkeln von 0˚ bis 25˚. Das dargestellte afokale Demonstrationsglas gemäß dieser Ausführungsform umfasst eine sphärische Vorderfläche mit einem Flächenbrechwert $D_1 = 6,0$ dpt und eine sphärische Rückfläche mit einem Flächenbrechwert $D_2 = -6,238$ dpt. Das Demonstrationsglas weist eine Mittendicke von $d_m = 10$mm und einen Brechungsindex von n = 1,502 auf.

Wie in Fig. 4A gezeigt bewirkt dieses Demonstrationsglas ohne Verkippung (Verkippungswinkel von 0˚) eine im wesentlichen konstante Vergrößerung von etwa 1,04. Die Linie konstanter Vergrößerung von genau 1,04 ist in Fig. 4A als nahezu kreisförmige Linie dargestellt. In einer bevorzugten Ausführungsform der Erfindung weist die Differenz der Vergrößerungswerte zwischen maximaler und minimaler Vergrößerung des Demonstrationsglases ohne Verkippung bei zumindest einer standardisierten Gebrauchsstellung einen Wert von weniger als 1% auf. Je nach Ausführungsform betrifft dies vorzugsweise jeweils zumindest eine der oben genannten standardisierten Gebrauchsstellungen und/oder die Eigenvergrößerung der Demonstrationsgläser. Fig. 4B zeigt Linien konstanter Verzerrung; wobei die dargestellten Linien Werte der Verzerrung von 1 % (geschlossene innere Linie) und 2% ohne Verkippung des Demonstrationsglases repräsentieren.

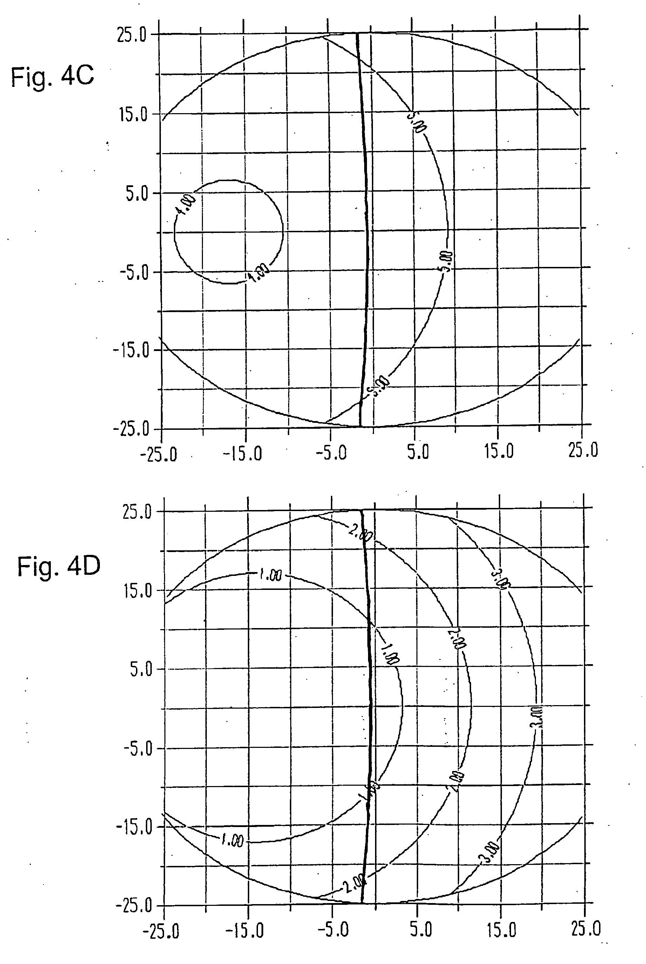

Durch Verkippen des Demonstrationsglases von Fig. 4A und 4B um 15˚ wird eine veränderte Vergrößerung des Glases erreicht, wie in Fig. 4C dargestellt. Die gezeigten Linien konstanter Vergrößerung repräsentieren Werte der Vergrößerung von 1,04 (geschlossene Linie) und 1,05. Fig. 4D zeigt die resultierenden Werte der Verzerrung des Demonstrationsglases bei der Verkippung von 15˚. Die gezeigten Linien repräsentieren jeweils Werte der Verzerrung von 1%, 2% bzw. 3%.

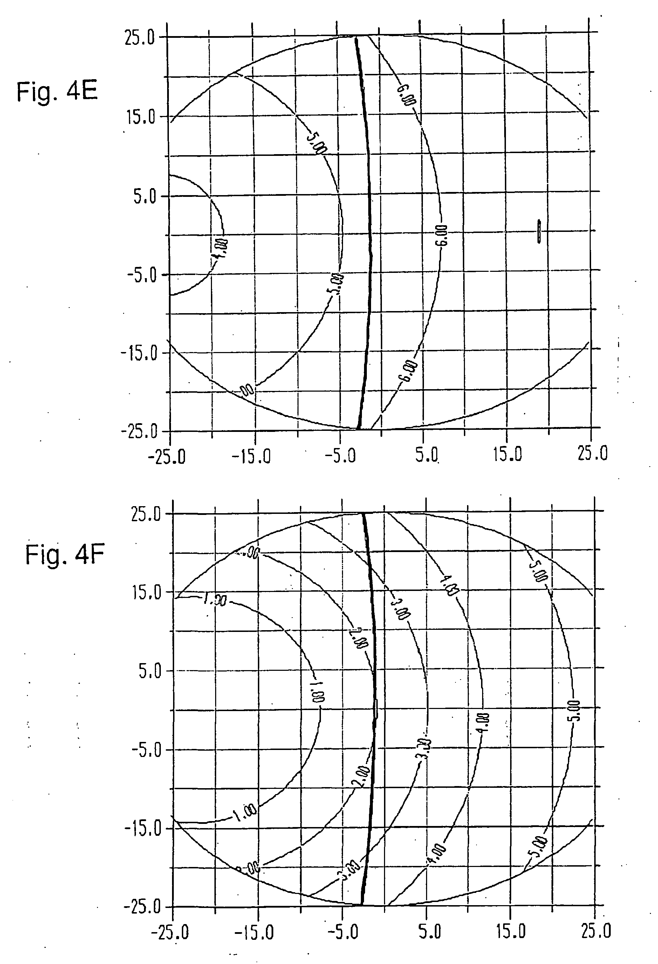

Durch eine weitere Verkippung des Demonstrationsglases kann, wie in Fig. 4E und 4F gezeigt, die Verteilung der Vergrößerung und der Verzerrung weiter verändert und insbesondere der Unterschied für den temporalen und nasalen Bereich weiter vergrößert werden. So steigt für dasselbe Demonstrationsglas bei einem Verkippungswinkel von 25˚ die Vergrößerung von einem Wert von etwa 1,04 im temporalen Bereich kontinuierlich auf einen Wert von fast 1,07 nahe dem nasalen Bereich des Demonstrationsglases. Die in Fig. 4E dargestellten Linien konstanter Vergrößerung repräsentieren jeweils Werte der Vergrößerung von 1,04; 1,05; 1,06 und 1,064. Wie in Fig. 4F gezeigt, steigt auch die Verzerrung vom temporalen zum nasalen Bereich kontinuierlich an. Insbesondere wird der Anstieg, d.h. der Gradient der Verzerrung durch die Erhöhung des Verkippungswinkels von 15˚ auf 25˚ vergrößert. Die in Fig. 4F dargestellten Linien konstanter Verzerrung repräsentieren jeweils Werte der Verzerrung von 1 % (im temporalen Bereich) bis 5% (im nasalen Bereich) in Schritten von 1%.

Somit lässt sich das dargestellte Demonstrationsglas unter einem geeigneten Verkippungswinkel vor zumindest ein Auge des Brillenträgers halten, um je nach Verkippungswinkel unterschiedliche Sportbrillengläser zu simulieren. Der Verkippungswinkel ebenso wie die Vergrößerung, also die Auswahl des Demonstrationsglases einer geeigneten Eigenvergrößerung wird in Abhängigkeit von den vorzugsweise analytisch berechneten optischen Werten der geplanten Sportbrille festgelegt. Mit dem vorgehaltenen Demonstrationsglas erhält der Brillenträger einen Eindruck von den zu erwartenden Vergrößerungen und/oder Verzerrungen sowie deren Verteilung über das Sichtfeld und kann bereits im Vorfeld erkennen, ob Unverträglichkeiten auftreten.

In einer weiteren bevorzugten Ausführungsform der Erfindung wird zumindest ein Demonstrationsglas mit zumindest einer sphärisch atorischen Fläche bereitgestellt. Als atorische Fläche wird in diesem Zusammenhang eine Fläche betrachtet, die zwei zueinander senkrechte Hauptschnitte unterschiedlicher Krümmung besitzt, und bei der der Schnitt durch mindestens einen der Hauptschnitte nicht kreisförmig ist. In diesem Zusammenhang wird insbesondere auf die Norm DIN EN ISO 13666 verwiesen. Als sphärisch atorische Fläche wird in diesem Zusammenhang ein Sonderfall einer atorischen Fläche betrachtet, bei der in einem Flächenreferenzpunkt, insbesondere im Scheitelpunkt, die beiden Hauptkrümmungen gleich sind, d.h. der Flächenastigmatismus verschwindet. Die sphärisch atorische Fläche hat demnach im Flächenreferenzpunkt, insbesondere im Scheitelpunkt, bei senkrechtem Einfall keine astigmatische, sondern nur eine sphärische Wirkung. Insbesondere verschwindet im Flächenreferenzpunkt, insbesondere im Scheitelpunkt, bei senkrechtem Einfall auch die Verzerrung. Beispielsweise könnte die Vorderfläche als sphärische Fläche und die Rückfläche als sphärisch atorische Fläche ausgestaltet sein. Vorzugsweise weist die Rückfläche temporal und nasal unterschiedliche Asphärizität auf. Damit können vorzugsweise nahezu beliebige, insbesondere in Sportbrillen auftretende Vergrößerungs- bzw. Verzerrungsverläufe zwischen nasalem und temporalem Bereich erreicht werden.

**Fig. 5A** bis **Fig. 5D** zeigen den Verlauf bzw. die Verteilung der Vergrößerung und der Verzerrung für ein solches afokales Demonstrationsglas mit einer sphärischen Vorderfläche mit einer Flächenbrechkraft $D_1$ = 6 dpt und einer sphärisch atorischen Rückfläche bei einem Verkippungswinkel von 15˚ (Fig. 5A und 5B) bzw. 25˚ (Fig. 5C und 5D). Das Demonstrationsglas weist in dieser bevorzugten Ausführungsform der Erfindung eine Mittendicke von $d_m$ = 10mm und einen Brechungsindex von n = 1,502 auf.

Fig. 5A zeigt die Vergrößerung des Demonstrationsglases in Abhängigkeit von der Durchblickstelle durch die Vorderfläche bei einem Verkippungswinkel von 15˚. Die dargestellten Linien repräsentieren von links nach rechts Werte konstanter Vergrößerung zwischen 1,03 und 1,08 in Stufen von 1%. Wie aus einem Vergleich mit Fig. 5C erkennbar ist, nimmt der Gradient der Vergrößerung mit steigendem Verkippungswinkel zu. So ist in Fig. 5C die Verteilung der Vergrößerung des Demonstrationsglases von Fig. 5A bei einem um 10˚ erhöhte Verkippungswinkel von insgesamt 25˚ gezeigt. Die dargestellten Linien repräsentieren von links nach rechts Werte konstanter Vergrößerung zwischen 1,03 und 1,10.

Die entsprechenden Verzerrungen für die beiden Verkippungswinkel von, 15˚ und 25˚ sind in Fig. 5B bzw. Fig. 5D dargestellt. Fig. 5B zeigt Linien konstanter Verzerrung, wobei die dargestellten Linien Werte der Verzerrung von 1 % (geschlossene innere Linie) bis 9% in Stufen von 1 % repräsentieren. Fig. 5D zeigt die Werte der Verzerrung des Demonstrationsglases bei der Verkippung von 15˚. Die gezeigten Linien repräsentieren jeweils Werte der Verzerrung von 1 % (innerste geschlossene Linie) bis 12% in Schritten von 1%.

In einem weiteren Aspekt der vorliegenden Erfindung wird ein Satz bzw. eine Serie von afokalen Messgläsern mit vorzugsweise meridional unterschiedlicher Vergrößerung, d.h. mit einer Verzerrung bereitgestellt.

Fig. 6A und 6B zeigen die Vergrößerung bzw. die Verzerrung in Abhängigkeit von der Durchblickrichtung bzw. -position eines beispielhaften Messglases aus einer Serie von afokalen Messgläsern gemäß einer bevorzugten Ausführungsform der vorliegenden Erfindung. Das gezeigte Messglas weist eine Vorderfläche mit senkrecht zueinander stehenden meridionalen Flächenbrechkräften von $D_{11}$ = 10 dpt bzw. $D_{12}$ = 5 dpt auf. Die Mittendicke des Messglases beträgt $d_m$ = 10 mm und der Brechungsindex liegt bei n = 1,502.

Wie in Fig. 6A dargestellt, weist das gezeigt Messglas eine im wesentlichen konstante Vergrößerung von 5% auf. Vorzugsweise variiert die Vergrößerung mit der Durchblickrichtung bzw. -position für jedes Messglas des Messgläsersatzes um weniger als 1%. Vorzugsweise variiert die Verzerrung mit der Durchblickrichtung bzw. -position für jedes Messglas des Messgläsersatzes um weniger als 2%. Dies ist insbesondere in Fig. 6B dargestellt. Die Verzerrung des gezeigten beispielhaften Messglases liegt in einem Bereich von etwa 3% bis 4%.

Vorzugsweise weisen die Messgläser torische Vorderflächen und torische Rückflächen auf. Mit solchen vorzugsweise bitorischen Gläsern in unterschiedlichen Verzerrungsstufen und eventuell rechts/links um 90˚ gedreht vorgehalten könnte der individuelle Schwellenwert für die Fusion anamorphotisch verzerrter Objekte und damit ein Verträglichkeitsgrenzwert für die Verzerrung und/oder den Verzerrungsunterschied zwischen beiden Augen des Brillenträgers bestimmt werden.

Das erfindungsgemäße Vorhalten von ausgewählten Gläsern aus einem erfindungsgemäßen Satz von Demonstrationsgläsern bzw. Messgläsern ist nicht nur für die Untersuchung bzw. Bestimmung der Verträglichkeit von Einstärkengläsern, sondern vorzugsweise bei allen Korrektionsgläsern, insbesondere auch bei Gleitsichtgläsern möglich. Dabei wird vorzugsweise zur gewohnten Abbildungscharakteristik mit der normalen Korrektion durch Vorhalten der ausgewählten Demonstrations- bzw. Messgläser die jeweils zu erwartenden und individuell unterschiedlich empfundenen Trageeigenschaften mit Sportbrillengläsern simuliert.

Die Vergrößerung in jedem Hauptmeridian kann sowohl für die Messgläser als auf für die Demonstrationsgläser mit folgenden Formeln analog zu zentrierten Einstärkengläsern berechnet werden:

$$N = N_S \cdot N_e$$

mit

$$N_S = \frac{1}{1 - e * S'_\infty}$$

und

$$N_e = \frac{1}{1 - \frac{d}{n} D_1} \qquad .$$

Dabei ist N die Gesamtvergrößerung bzw. Vergrößerung für den Brillenträger, $N_S$ die Systemvergrößerung, $N_e$ die Eigenvergrößerung des Brillenglases bzw. Demonstrationsglases bzw. Messglases, d die Mittendicke, $D_1$ der Vorderflächenbrechwert, $S'_\infty$ der Scheitelbrechwert, n die Brechzahl und e* der Hauptpunkt-Scheitelabstand. Wird ein Demonstrationsglas oder ein Messglas zusätzlich zu einer gewohnten Brille des Brillenträgers genutzt, sind für eine Bestimmung der Vergrößerung entsprechend beide Komponenten zu berücksichtigen. Für weitere Details zur Bestimmung von Vergrößerungen wird wiederum ausdrücklich auf das Fachbuch von Heinz Diepes und Ralf Blendowske "Optik und Technik der Brille", Ausgabe 2002, Optische Fachveröffentlichung GmbH, Heidelberg und die Druckschrift DE 10 2005 057 533 A1 Bezug genommen.

Für die Bedingung $S'_\infty = 0$ wird die Systemvergrößerung $N_S$ zu 1 und die beiden Hauptvergrößerungen $N_1$ und $N_2$ können über die Vorgabe von $D_{11}$ im ersten Hauptschnitt und $D_{12}$ im zweiten Hauptschnitt mit der Formel der Eigenvergrößerung bestimmt werden. Die Rückflächenbrechwerte sind durch die Vorgabe des Scheitelbrechwertes dann eindeutig bestimmt.

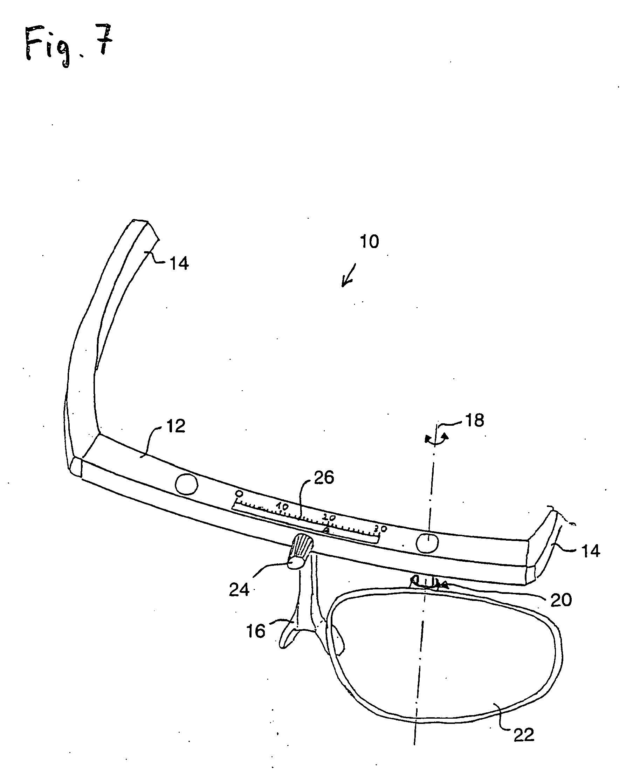

In einer bevorzugten Ausführungsform wird zum Vorhalten des zumindest einen ausgewählten Demonstrationsglases eine Demonstrationsfassung bzw. Demonstrationsbrillenfassung genutzt, in die das Demonstrationsglas einsetzbar ist. **Fig. 7** zeigt eine beispielhafte Demonstrationsfassung 10 gemäß einer bevorzugten Ausführungsform. Die Demonstrationsfassung 10 umfasst eine Fassungsschiene 12, an deren Enden jeweils ein Bügel 14 angeordnet ist. Über diese Bügel 14 kann die Demonstrationsfassung vorzugsweise an den Ohren des Brillenträgers abgestützt werden. In einer anderen, nicht gezeigten Ausführungsform umfasst die Demonstrationsfassung zusätzlich zu oder anstatt der Bügel 14 Befestigungselemente wie z.B. eine Fassungshalterung zum Befestigen oder Abstützen der Demonstrationsfassung an einer Brille des Brillenträgers. Außerdem weist die dargestellte Demonstrationsfassung 10 eine Nasenauflage 16 auf, mittels der die Demonstrationsfassung auf der Nase des Brillenträgers abgestützt werden kann. Vorzugsweise ist die Nasenauflage 16 in der Höhe einstellbar.

In der dargestellten bevorzugten Ausführungsform umfasst die Demonstrationsfassung 10 zumindest ein um eine Verkippungsachse 18 drehbares Montageelement bzw. Drehelement 20 über das ein Demonstrationsglas 22 drehbar und abnehmbar bzw. austauschbar an die Demonstrationsfassung 10 montiert werden kann. Ein gewünschter Verkippungswinkel des zumindest einen Demonstrationsglases lässt sich vorzugsweise über ein Betätigungselement 24 an der Demonstrationsfassung 10 einstellen. Eine Übertragung einer Drehbewegung vom Betätigungselement 24 zum Montageelement bzw. Drehelement 20 erfolgt vorzugsweise über ein Schneckengewinde, das in bzw. an der Fassungsschiene 12 vorgesehen ist. Zur Überprüfung und Einstellung des Verkippungswinkels weist die Demonstrationsfassung vorzugsweise außerdem eine Anzeigeeinrichtung 26, beispielsweise in Form einer Ableseskala auf, an der der eingestellte Verkippungswinkel abgelesen werden kann. Vorzugsweise ist das zumindest eine Demonstrationsglas 22 ausgehend von einer Demonstrationsausgangsstellung, welche vorzugsweise einem Fassungsscheibenwinkel von 0° entspricht, um zumindest 25° nach außen, also zu zunehmenden Fassungsscheibenwinkeln hin verkippbar.

Vorzugsweise weist die Demonstrationsfassung zwei Montageelemente bzw. Drehelemente 20 auf, über die jeweils ein rechtes bzw. ein linkes Demonstrationsglas 22 drehbar und abnehmbar an die Demonstrationsfassung 10 montiert werden kann. Beide Montage- bzw. Drehelemente und Demonstrationsgläser sind mittels des Betätigungselements vorzugsweise synchron und spiegelsymmetrisch zueinander verkippbar. Damit ist der Scheibenwinkel der beiden Demonstrationsgläser vorzugsweise stets gleich groß. In einer nicht dargestellten bevorzugten Ausführungsform ist der gegenseitige Abstand der beiden Montageelemente und damit der Abstand der beiden Verkippungsachsen zueinander einstellbar, um die Demonstrationsfassung an die individuelle Pupillendistanz des Brillenträgers anpassen zu können.

Insgesamt kann durch die vorliegende Erfindung einem Benutzer, wie z.B. einem Augenoptiker oder Sportgeschäft etc., ein Verfahren und ein Computerprogramm an die Hand gegeben werden, mit dessen Hilfe über die Glasstärke des Kunden errechnet werden kann, welche Vergrößerungen und/oder Verzerrungen bei welcher Verkippung der Brillengläser (oder z.B. bei einer bestimmten Verkippung bei einer bereits ausgewählten Fassung) zu erwarten wären. Mit Hilfe der geeigneten Demonstrationsgläser aus einem bereitgestellten Satz von Demonstrationsgläsern könnte nun dem Kunden die zu erwartende Abweichung vom Seheindruck mit der normalen Brille demonstriert werden. Damit können eventuelle Verträglichkeitsprobleme bereits vor einer Fertigung oder Bestellung einer Sportbrille bereits erkannt werden. Außerdem kann mit Hilfe eines Satzes von Messgläsern (evtl. gleich im Zuge der Refraktion) gemessen werden, wie groß die Veränderung der Abbildungseigenschaften, die der Kunde noch als tolerierbar empfindet, sein dürfte. Nun könnte er über die Berechnung der Abbildungseigenschaften (unter Eingabe von Fehlsichtigkeit und Fassungsscheibenwinkel der gewählten Fassung) herausfinden, wie groß die für den Kunden zu erwartenden "angenehmen/unproblematischen" Sehbereiche wären. Sind diese Bereich groß, so ist zu erwarten, dass der Kunde die fertige Brille als verträglich empfindet. Ergeben sich nur kleine Bereiche, bei denen eine Akzeptanz der veränderten Vergrößerung und/oder Verzerrung zu erwarten ist, dann wird die fertige Brille mehr Umstellungsprobleme verursachen.

**Bezugszeichenliste**

| | |
|---|---|
| 2 | Demonstrationsglas |
| 4 | Auge |
| 6 | Vorderfläche |
| 8 | Rückfläche |
| 10 | Demonstrationsfassung |
| 12 | Fassungsschiene |
| 14 | Bügel |
| 16 | Nasenauflage |
| 18 | Verkippungsachse |
| 20 | drehbares Montageelement bzw. Drehelement |
| 22 | austauschbares Demonstrationsglas |
| 24 | Betätigungseinrichtung |
| 26 | Anzeigeeinrichtung |
| β | Verkippungswinkel |
| Z' | Augendrehpunkt |

**Patentansprüche**

1. Verfahren zur Untersuchung der Verträglichkeit einer Brille mit großem Fassungsscheibenwinkel und/oder mit starker Durchmuschelung für einen Brillenträger, umfassend:

   - Entwerfen zumindest eines Brillenglases der Brille für den Brillenträger;
   - Ermitteln einer Vergrößerung und/oder einer Verzerrung des zumindest einen Brillenglases für zumindest eine Blickrichtung des Brillenträgers;
   - Auswählen desjenigen afokalen Demonstrationsglases mit einer bestimmten Eigenvergrößerung aus einer Serie von afokalen Demonstrationsgläsern mit zumindest teilweise unterschiedlichen Eigenvergrößerungen, das in einer Demonstrationsstellung vor dem entsprechenden Auge des Brillenträgers für die zumindest eine Blickrichtung des Brillenträgers zu einer Vergrößerung und/oder Verzerrung führt, die der ermittelten Vergrößerung bzw. Verzerrung des zumindest einen Brillenglases am nächsten kommt;
   - Vorhalten des ausgewählten afokalen Demonstrationsglases in der Demonstrationsstellung vor das entsprechende Auge des Brillenträgers.

2. Verfahren nach Anspruch 1, außerdem umfassend ein Bestimmen eines Verkippungswinkels für das zumindest eine ausgewählte afokale Demonstrationsglas abhängig von der ermittelten Vergrößerung und/oder Verzerrung des zumindest einen Brillenglases, wobei das Vorhalten des zumindest einen ausgewählten Demonstrationsglases ein Vorhalten des Demonstrationsglases unter dem bestimmten Verkippungswinkel umfasst.

3. Verfahren nach einem der vorangegangenen Ansprüche, wobei das zumindest eine Demonstrationsglas zusätzlich zu einer gewohnten Brille des Brillenträger in der Demonstrationsstellung vor den Augen des Brillenträgers angeordnet wird.

4. Verfahren zur Bestimmung der Verträglichkeit einer Brille mit großem Fassungsscheibenwinkel und/oder starker Durchmuschelung für einen Brillenträger, umfassend:

   - Bereitstellen einer Serie von afokalen Messgläsern mit zumindest teilweise unterschiedlicher Eigenvergrößerung und/oder Verzerrung;
   - Bestimmen zumindest eines Verträglichkeitsgrenzwerts für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers mittels der Serie von afokalen Messgläsern;
   - Entwerfen zumindest eines Brillenglases der Brille für den Brillenträger; und
   - Ermitteln zumindest eines Akzeptanzbereichs des zumindest einen entworfenen Brillenglases als einen Flächenbereich des Brillenglases und/oder einen Blickwinkelbereich des Brillenträgers in Abhängigkeit von dem zumindest einen bestimmten Verträglichkeitsgrenzwert.

5. Verfahren nach Anspruch 4, wobei das Ermitteln eines Akzeptanzbereichs umfasst:

   - Ermitteln von Vergrößerungen und/oder Verzerrungen des zumindest einen Brillenglases und/oder von Vergrößerungsunterschieden und/oder Verzerrungsunterschieden zwischen beiden Brillengläsern der Brille für eine Vielzahl von Blickrichtungen des Brillenträgers; und
   - Auswählen von Blickrichtungen für die die ermittelte Vergrößerung und/oder Verzerrung bzw. der ermittelte Vergrößerungs- und/oder Verzerrungsunterschied nicht über dem zumindest einen bestimmten Verträglichkeitsgrenzwert liegt.

6. Computersystem zur Untersuchung der Verträglichkeit einer Brille mit großem Fassungsscheibenwinkel und/oder mit starker Durchmuschelung für einen Brillenträger, umfassend:

   - ein Entwurfsmodul zum Entwerfen zumindest eines Brillenglases der Brille für den Brillenträger;
   - eine Analyseeinrichtung zum Ermitteln einer Vergrößerung und/oder einer Verzerrung des entworfenen Brillenglases für zumindest eine Blickrichtung des Brillenträgers; und
   - eine Auswahleinrichtung zum Auswählen desjenigen afokalen Demonstrationsglases mit einer bestimmten Eigenvergrößerung aus einer Serie von afokalen Demonstrationsgläsern mit zumindest teilweise unterschiedlichen Eigenvergrößerungen, das in einer Demonstrationsstellung vor dem entsprechenden Auge des Brillenträgers für die zumindest eine Blickrichtung des Brillenträgers zu einer Vergrößerung und/oder Verzerrung führt, die der ermittelten Vergrößerung bzw. Verzerrung des zumindest einen Brillenglases am nächsten kommt, für ein Vorhalten des ausgewählten afokalen Demonstrationsglases in der Demonstrationsstellung vor das entsprechende Auge des Brillenträgers.

7. Computersystem zur Bestimmung der Verträglichkeit einer Brille mit großem Fassungsscheibenwinkel und/oder starker Durchmuschelung für einen Brillenträger, umfassend:

- eine Erfassungseinrichtung zum Erfassen zumindest eines Verträglichkeitsgrenzwertes für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers;
- ein Entwurfsmodul zum Entwerfen zumindest eines Brillenglases der Brille für den Brillenträger;
- eine Analyseeinrichtung zum Ermitteln eines Akzeptanzbereichs des zumindest einen entworfenen Brillenglases als einen Flächenbereich des Brillenglases und/oder einen Blickwinkelbereich des Brillenträgers in Abhängigkeit von dem zumindest einen erfassten Verträglichkeitsgrenzwert; und
- eine Anzeigeeinrichtung zur graphischen Darstellung eines den ermittelten Akzeptanzbereich des entworfenen Brillenglases repräsentierenden Flächen- und/oder Blickwinkelbereichs.

8. Computerimplementiertes Verfahren zur Untersuchung der Verträglichkeit einer Brille mit großem Fassungsscheibenwinkel und/oder mit starker Durchmuschelung für einen Brillenträger bereit, umfassend:

- Entwerfen zumindest eines Brillenglases der Brille für den Brillenträger;
- Ermitteln einer Vergrößerung und/oder einer Verzerrung des zumindest einen Brillenglases für zumindest eine Blickrichtung des Brillenträgers; und
- Auswählen desjenigen afokalen Demonstrationsglases mit einer bestimmten Eigenvergrößerung aus einer Serie von afokalen Demonstrationsgläsern mit zumindest teilweise unterschiedlichen Eigenvergrößerungen, das in einer Demonstrationsstellung vor dem entsprechenden Auge des Brillenträgers für die zumindest eine Blickrichtung des Brillenträgers zu einer Vergrößerung und/oder Verzerrung führt, die der ermittelten Vergrößerung bzw, Verzerrung des zumindest einen Brillenglases am nächsten kommt, für ein Vorhalten des ausgewählten afokalen Demonstrationsglases in der Demonstrationsstellung vor das entsprechende Auge des Brillenträgers.

9. Computerimplementiertes Verfahren zur Bestimmung der Verträglichkeit einer Brille mit großem Fassungsscheibenwinkel und/oder starker Durchmuschelung, insbesondere einer Sportbrille, für einen Brillenträger, umfassend:

- Erfassen zumindest eines Verträglichkeitsgrenzwertes für eine Vergrößerung und/oder eine Verzerrung in Bezug auf zumindest ein Auge des Brillenträgers und/oder für einen Vergrößerungsunterschied und/oder einen Verzerrungsunterschied zwischen den beiden Augen des Brillenträgers;
- Entwerfen zumindest eines Brillenglases der Brille für den Brillenträger;
- Ermitteln eines Akzeptanzbereichs des zumindest einen entworfenen Brillenglases als einen Flächenbereich des Brillenglases und/oder einen Blickwinkelbereich des Brillenträgers in Abhängigkeit von dem zumindest einen erfassten Verträglichkeitsgrenzwert; und
- graphisches Darstellen eines den ermittelten Akzeptanzbereich des entworfenen Brillenglases repräsentierenden Flächen- und/oder Blickwinkelbereichs.

10. Computerprogrammprodukt, umfassend Programmcode, der, wenn er in einem Computersystem geladen ist, dieses veranlasst ein Verfahren nach Anspruch 8 oder 9 auszuführen.

11. Demonstrationsgläsersatz zur Durchführung des Verfahrens von Anspruch 1 umfassend eine Vielzahl von für zumindest eine spezielle Demonstrationsausgangsstellung ausgelegten Demonstrationsgläsern derart, dass die Demonstrationsgläser im wesentlichen keine dioptrische Wirkung, zumindest teilweise unterschiedliche Eigenvergrößerung und in der zumindest einen Demonstrationsausgangsstellung für zumindest eine Durchblickrichtung im wesentlichen keine Verzerrung aufweisen.

12. Demonstrationsgläsersatz nach Anspruch 11, wobei die zumindest teilweise unterschiedlichen Eigenvergrößerungen der Demonstrationsgläser in einem Bereich zwischen 1,01 und 1,1, vorzugsweise in einem Bereich zwischen 1,02 und 1,06 liegen.

13. Demonstrationsgläsersatz nach Anspruch 11 oder 12, wobei die Demonstrationsgläser eine Mittendicke im Bereich zwischen 2 mm und 20 mm, vorzugsweise zwischen 5 mm und 15 mm aufweisen.

**14.** Messgläsersatz zur Durchführung des Verfahrens von Anspruch 4 umfassend eine Vielzahl von für zumindest eine Verträglichkeitsmessstellung ausgelegten Messgläsern derart, dass die Messgläser im wesentlichen keine dioptrische Wirkung, eine im wesentlichen konstante Eigenvergrößerung und in der zumindest einen Verträglichkeitsmessstellung zumindest teilweise unterschiedliche Verzerrung aufweisen.

**15.** Messgläsersatz nach Anspruch 14, wobei zumindest eine Vielzahl der Messgläser bitorische Gläser sind.

**Claims**

**1.** Method for examining the tolerability of spectacles with a large frame lens angle and/or with high curvature for a spectacle wearer, comprising:

- designing at least one spectacle lens of the spectacles for the spectacle wearer;
- determining a magnification and/or a distortion of the at least one spectacle lens for at least one viewing direction of the spectacle wearer;
- selecting the afocal demonstration lens with a specific inherent magnification from a series of afocal demonstration lenses with at least partially different inherent magnifications, which in a demonstration position in front of the corresponding eye of the spectacle wearer for the at least one viewing direction of the spectacle wearer leads to a magnification and/or distortion, which comes closest to the determined magnification or distortion of the at least one spectacle lens;
- holding the selected afocal demonstration lens in the demonstration position in front of the corresponding eye of the spectacle wearer.

**2.** Method according to claim 1, further comprising a determination of a tilt angle for the at least one selected afocal demonstration lens depending on the determined magnification and/or distortion of the at least one spectacle lens, wherein the holding of the at lest one selected demonstration lens comprises a holding of the demonstration lens at the specific tilt angle.

**3.** Method according to one of the preceding claims, wherein the at least one demonstration lens is arranged in the demonstration position in front of the eyes of the spectacle wearer in addition to customary spectacles of the spectacle wearer.

**4.** Method for determining the tolerability of spectacles with a large frame lens angle and/or with high curvature for a spectacle wearer, comprising:

- providing a series of afocal measurement lenses with at least partially different inherent magnification and/or distortion;
- determining at least one tolerability limit value for a magnification and/or a distortion in relation to at least one eye of the spectacle wearer and/or for a magnification difference and/or a distortion difference between the two eyes of the spectacle wearer by means of the series of afocal measurement lenses;
- designing at least one spectacle lens of the spectacles for the spectacle wearer; and
- determining at least one acceptance range of the at least one designed spectacle lens as a surface region of the spectacle lens and/or a viewing angle range of the spectacle wearer in dependence on the at least one specific tolerability limit value.

**5.** Method according to claim 4, wherein the determination of an acceptance range comprises:

- determining magnifications and/or distortions of the at least one spectacle lens and/or of magnification differences and/or distortion differences between two spectacle lenses of the spectacles for a plurality of viewing directions of the spectacle wearer; and
- selecting viewing directions for the determined magnification and/or distortion or the determined magnification and/or distortion difference does not lie above the at least one specific tolerability limit value.

**6.** Computer system for examining the tolerability of spectacles with a large frame lens angle and/or with high curvature for a spectacle wearer, comprising:

● a design module for designing at least one spectacle lens of the spectacles for the spectacle wearer;

● an analysis arrangement for determining a magnification and/or a distortion of the designed spectacle lens for at least one viewing direction of the spectacle wearer; and

● a selection arrangement for selecting the afocal demonstration lens with a specific inherent magnification from a series of afocal demonstration lenses with at least partially different inherent magnifications, which in a demonstration position in front of the corresponding eye of the spectacle wearer for the at least one viewing direction of the spectacle wearer leads to a magnification and/or distortion, which comes closest to the determined magnification or distortion of the at least one spectacle lens, for holding the selected afocal demonstration lens in the demonstration position in front of the corresponding eye of the spectacle wearer.

7. Computer system for determining the tolerability of spectacles with a large frame lens angle and/or with high curvature for a spectacle wearer, comprising:

● an acquisition arrangement for acquiring at least one tolerability limit value for a magnification and/or a distortion in relation to at least one eye of the spectacle wearer and/or for a magnification difference and/or a distortion difference between the two eyes of the spectacle wearer;

● a design module for designing at least one spectacle lens of the spectacles for the spectacle wearer;

● an analysis arrangement for determining at least one acceptance range of the at least one designed spectacle lens as a surface region of the spectacle lens and/or a viewing angle range of the spectacle wearer in dependence on the at least one determined tolerability limit value; and

● a display arrangement for the graphic display of a surface region and/or viewing angle range representing the determined acceptance range of the designed spectacle lens.

8. Computer-implemented method for examining the tolerability of spectacles with a large frame lens angle and/or with high curvature for a spectacle wearer, comprising:

● designing at least one spectacle lens of the spectacles for the spectacle wearer;

● determining a magnification and/or a distortion of the at least one spectacle lens for at least one viewing direction of the spectacle wearer; and

● selecting the afocal demonstration lens with a specific inherent magnification from a series of afocal demonstration lenses with at least partially different inherent magnifications, which in a demonstration position in front of the corresponding eye of the spectacle wearer for the at least one viewing direction of the spectacle wearer leads to a magnification and/or distortion, which comes closest to the determined magnification or distortion of the at least one spectacle lens for holding the selected afocal demonstration lens in the demonstration position in front of the corresponding eye of the spectacle wearer.

9. Computer-implemented method for determining the tolerability of spectacles with a large frame lens angle and/or with high curvature, in particular sports eyewear, for a spectacle wearer, comprising:

● determining at least one tolerability limit value for a magnification and/or a distortion in relation to at least one eye of the spectacle wearer and/or for a magnification difference and/or for a distortion difference between the two eyes of the spectacle wearer;

● designing at least one spectacle lens of the spectacles for the spectacle wearer;

● determining an acceptance range of the at least one designed spectacle lens as a surface region of the spectacle lens and/or a viewing angle range of the spectacle wearer in dependence on the at least one determined tolerability limit value; and

● graphically displaying a surface region and/or viewing angle range representing the determined acceptance range of the designed spectacle lens.

10. Computer program product comprising program code, which when loaded on a computer system causes this to conduct a method according to claim 8 or 9.

11. Demonstration lens set for conducting the method of claim 1, comprising a plurality of demonstration lenses designed for at least one special demonstration starting position such that the demonstration lenses have substantially no dioptric effect, at least partially different inherent magnification and in the at least one demonstration starting position have substantially no distortion for at least one viewing direction.

12. Demonstration lens set according to claim 11, wherein the at least partially different inherent magnifications of the demonstration lenses lie in a range of between 1.01 and 1.1, preferably in a range of between 1.02 and 1.06.

13. Demonstration lens set according to claim 11 or 12, wherein the demonstration lenses have a centre thickness in the range of between 2 mm and 20 mm, preferably between 5 mm and 15 mm.

14. Measurement lens set for conducting the method of claim 1, comprising a plurality of measurement lenses designed for at least one tolerability measurement position such that the measurement lenses have substantially no dioptric effect, a substantially constant inherent magnification and in the at least one tolerability measurement position have at least partially different distortion.

15. Measurement lens set according to claim 14, wherein at least a plurality of the measurement lenses are bitoric lenses.

**Revendications**

1. Procédé pour l'étude de la compatibilité d'une paire de lunettes comportant un angle d'inclinaison de monture élevé et/ou une forte courbure globale pour un porteur de lunettes comprenant :

   - la conception d' au moins un verre de lunette de la paire de lunettes pour le porteur de lunettes ;
   - la détermination d'un grossissement et/ou d'une déformation d'au moins un verre de lunettes pour au moins un sens d'observation du porteur de lunettes ;
   - la sélection du verre de démonstration afocal avec un grossissement spécifique déterminé à partir d'une série de verres de démonstration afocaux avec au moins partiellement des grossissements spécifiques différents, qui, dans une position de démonstration devant l'oeil correspondant du porteur de lunettes pour au moins un sens d'observation du porteur de lunettes, conduit à un grossissement et/ou à une déformation, qui se rapproche le plus du grossissement, respectivement de la déformation, déterminés pour au moins un verre de lunettes ;
   - la présentation du verre de démonstration afocal sélectionné dans la position de démonstration devant l'oeil correspondant du porteur de lunettes.

2. Procédé selon la revendication 1 comprenant en outre la détermination d'un angle de basculement pour au moins un grossissement déterminé et/ou une déformation déterminée d'au moins un verre de lunettes en fonction du verre de démonstration au moins sélectionné, la présentation du verre de démonstration au moins sélectionné comprenant une présentation du verre de démonstration sous un angle de basculement déterminé.

3. Procédé selon l'une des revendications précédentes où au moins un verre de démonstration est disposé, en plus d'une paire de lunettes usuelle du porteur de lunettes, dans une position de démonstration devant les yeux du porteur de lunettes.

4. Procédé pour la détermination de la compatibilité d'une paire de lunettes avec un angle d'inclinaison de monture élevé et/ou avec une forte courbure globale pour un porteur de lunettes, comprenant :

   - la mise à disposition d'une série de verres de mesure afocaux avec au moins partiellement des grossissements spécifiques et/ou des déformations différents ;
   - la détermination d'au moins une valeur limite de compatibilité pour un grossissement et/ou une déformation en ce qui concerne au moins un oeil du porteur de lunettes, et/ou pour une différence de grossissement et/ou pour une différence de déformation entre les deux yeux du porteur de lunettes au moyen d'une série de verres de mesure afocaux ;
   - la conception d'au moins un verre de lunettes de la paire de lunettes pour le porteur de lunettes ; et
   - la détermination d'au moins un domaine d'acceptation pour au moins un verre de lunettes élaboré en tant que partie de la surface du verre de lunette et/ou que domaine d'angle de vision du porteur de lunettes en fonction d'au moins une valeur limite de compatibilité déterminée.

5. Procédé selon la revendication 4 où la détermination d'un domaine d'acceptation comprend :

   - la détermination des grossissements et/ou des déformations d'au moins un verre de lunettes, et/ou des différences de grossissements et/ou des différences de déformations entre deux verres de lunettes de la paire de lunettes pour une multitude de sens d'observation du porteur de lunettes ; et
   - la sélection de sens d'observation pour le grossissement déterminé et/ou la déformation d'au moins un verre de lunettes, respectivement pour la différence de grossissement déterminée et/ou pour la différence de déformation au dessus desquels se situe une valeur limite de compatibilité déterminée.

**6.** Système par ordinateur pour l'examen de la compatibilité d'une paire de lunettes avec un angle d'inclinaison de monture élevé et/ou avec une forte courbure globale pour un porteur de lunettes, comprenant :

- un module de conception pour concevoir au moins un verre de lunettes de la paire de lunettes pour le porteur de lunettes ;
- une unité d'analyse pour l'évaluation d'un grossissement et/ou d'une déformation du verre de lunettes élaboré pour au moins un sens d'observation du porteur de lunettes ; et
- une unité de sélection pour la sélection du verre de démonstration afocal comprenant un grossissement spécifique déterminé à partir d'une série de verres de démonstration afocaux avec au moins partiellement des grossissements spécifiques différents, verre qui, dans une position de démonstration devant l'oeil correspondant du porteur de lunettes, conduit à un grossissement et/ou à une déformation dans au moins un d'observation du porteur de lunettes, qui se rapproche le plus au moins d'un verre de lunettes pour une présentation du verre de démonstration afocal sélectionné dans une position de démonstration devant l'oeil correspondant du porteur de lunettes.

**7.** Système par ordinateur pour la détermination de la compatibilité d'une paire de lunettes comprenant un angle d'inclinaison de monture élevé et/ou une forte courbure globale pour un porteur de lunettes, comprenant :

- une unité d'évaluation pour l'évaluation d'au moins une valeur limite de compatibilité pour un grossissement et/ou une déformation en ce qui concerne au moins un oeil du porteur de lunettes, et/ou pour une différence de grossissement et/ou une différence de déformation entre les deux yeux du porteur de lunettes ;
- un module de conception pour la conception d'au moins un verre de lunettes de la paire de lunettes pour le porteur de lunettes ;
- une unité d'analyse pour l'évaluation d'un domaine d'acceptation pour au moins un verre de lunettes élaboré en tant que partie de surface du verre de lunettes et/ou que domaine d'angle de vision du porteur de lunettes en fonction d' au moins une valeur limite de compatibilité évaluée ; et
- une unité de visualisation pour la représentation graphique d'un domaine d'acceptation évalué en tant que partie de surface et/ou de domaine d'angle de vision représentant le verre de lunettes élaboré.

**8.** Procédé implémenté par ordinateur pour l'étude de la compatibilité d'une paire de lunettes possédant un angle d'inclinaison de monture élevé et/ou une forte courbure globale pour un porteur de lunettes, comprenant .

- la conception d'au moins un verre de lunettes de la paire de lunettes pour le porteur de lunettes ;
- la détermination d'un grossissement et/ou d'une déformation d'au moins un verre de lunettes pour au moins un sens d'observation du porteur de lunettes ; et
- la sélection du verre de démonstration comprenant un grossissement spécifique déterminé à partir d'une série de verres de démonstration afocaux avec au moins partiellement des grossissements spécifiques différents, qui, dans une position de démonstration devant l'oeil correspondant du porteur de lunettes pour au moins un sens d'observation du porteur de lunettes, aboutit à un grossissement et/ou à une déformation qui se rapproche le plus du grossissement, respectivement de la déformation, évalué pour au moins un verre de lunettes, pour une représentation du verre de démonstration afocal sélectionné dans la position de démonstration devant l'oeil correspondant du porteur de lunettes.

**9.** Procédé implémenté par ordinateur pour la détermination de la compatibilité d'une paire de lunettes possédant un angle d'inclinaison de monture élevé et/ou une forte courbure globale, en particulier des lunettes de sport pour un porteur de lunettes, comprenant :

- l'évaluation d'au moins une valeur limite de compatibilité pour un grossissement et/ou une déformation en ce qui concerne au moins un oeil du porteur de lunettes, et/ou pour une différence de grossissement et/ou pour une différence de déformation entre les deux yeux d'un porteur de lunettes ;
- la conception d'au moins un verre de lunettes de la paire de lunettes pour le porteur de lunettes ;
- la détermination d'un domaine d'acceptation d'au moins un verre de lunettes élaboré en tant que partie de surface du verre de lunettes et/ou que domaine d'angle de vision du porteur de lunettes pour au moins une valeur limite de compatibilité évaluée ; et
- la représentation graphique d'un domaine d'acceptation déterminé des parties de surfaces et/ou des domaines d'angle de vision représentant le verre de lunettes élaboré.

10. Produit d'un programme d'ordinateur comprenant un code de programme qui, lorsqu'il est chargé dans un système informatique, engage celui-ci à effectuer un procédé selon les revendications 8 ou 9.

11. Substitut de verre de démonstration pour la réalisation du procédé selon la revendication 1 comprenant une multitude de verres de démonstration employés pour une représentation de démonstration au moins spéciale, de telle sorte que les verres de démonstration employés ne présentent pas dans l'ensemble d'effet de dioptrie, présentent au moins partiellement des grossissements spécifiques différents et ne présentent pas dans l'ensemble de déformation dans au moins une représentation finale de démonstration pour au moins un sens d'observation.

12. Substitut de verre de démonstration selon la revendication 11, les grossissements spécifiques au moins partiellement différents des verres de démonstration se situant dans un domaine entre 1,01 et 1,1, de préférence dans un domaine entre 1,02 et 1,06.

13. Substitut de verre de démonstration selon les revendications 11 ou 12, les verres de démonstration présentant une épaisseur au centre dans l'intervalle entre 2 mm et 20 mm, de préférence entre 5 mm et 15 mm.

14. Substitut d'un verre de mesure pour la réalisation du procédé selon la revendication 4 comprenant une multitude de verres de mesure employés pour au moins une position de mesure de la compatibilité de sorte que les verres de mesure ne présentent dans l'ensemble aucun effet de dioptrie, présentent un grossissement spécifique dans l'ensemble contant et présentent au moins partiellement une déformation différente dans au moins une position de mesure de compatibilité.

15. Substitut de verre de mesure selon la revendication 14 où au moins une multitude des verres de mesure sont des verres bitoriques.

Fig. 1

EP 2 132 593 B1

Fig. 2A

Fig. 2B

Fig. 2C

Fig. 2D

Fig. 3A

Fig. 3B

Fig. 3C

Fig. 3D

Fig. 4A

Fig. 4B

Fig. 4C

Fig. 4D

Fig. 4E

Fig. 4F

Fig. 5A

Fig. 5B

Fig. 5C

Fig. 5D

Fig. 6A

Fig. 6B

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

*   DE 102005057533 A1

**In der Beschreibung aufgeführte Nicht-Patentliteratur**

*   Optik und Technik der Brille. **von Heinz Diepes ; Ralf Blendowske.** Optische Fachveröffentlichung GmbH. 2002